# EUROPEAN PATENT SPECIFICATION

(11) **EP 1 007 713 B1**
(45) Date of publication and mention of the grant of the patent: **15.06.2005**
(21) Application number: 98901377.6
(22) Date of filing: 26.01.1998
(51) Int. Cl.: C12N 15/85, C12N 15/62

(54) **DNA EXPRESSION IN TRANSFECTED CELLS AND ASSAYS CARRIED OUT IN TRANSFECTED CELLS**
DNA EXPRESSION IN TRANSFIZIERTEN ZELLEN UND TESTVERFAHREN IN TRANSFIZIEREEN ZELLEN
EXPRESSION DE L'ADN DANS DES CELLULES TRANSFECTEES ET DOSAGES EFFECTUES DANS DES CELLULES TRANSFECTEES

(30) Priority: 24.01.1997 GB 9701492
(43) Date of publication of application: 14.06.2000
(73) Proprietor: THE UNIVERSITY OF EDINBURGH, South Bridge, Edinburgh EH8 9YL (GB)
(72) Inventor: BLACKBURN, Catherine Claire, Univers.of Edinburgh, Edinburgh EH9 3JQ (GB); CHAMBERS, Ian, Paul, The University of Edinburgh, Edinburgh EH9 3JQ (GB); MEDVINSKY, Alexander, L., The Univ. of Edinburgh, Edinburgh EH9 3JQ (GB); NIWA, Hitoshi, The University of Edinburgh, Edinburgh EH9 3JQ (GB); SMITH, Austin, Gerard, The Univ. of Edinburgh, Edinburgh EH9 3JQ (GB)
(74) Representative: Schlich, George William
(86) International application number: PCT/GB1998/000216
(87) International publication number: WO 1998/032868

(56) References cited:
- EP-A- 0 731 169
- WO-A-96/40904
- M. GASSMANN ET AL.: "Maintenance of an extrachromosomal plasmid vector in mouse embryonic stem cells" PROCEEDINGS OF THE NATIONAL ACADEMY OF SCIENCES OF USA, vol. 92, no. 5, 28 February 1995, pages 1292-1296, XP002064504 WASHINGTON US cited in the application
- G. CAMENISCH ET AL.: "A polyoma-based episomal vector efficiently expresses exogenous genes in mouse embryonic stem cells" NUCLEIC ACIDS RESEARCH, vol. 24, no. 19, 1 October 1996, pages 3707-3713, XP002064505 OXFORD GB cited in the application
- P. MOUNTFORD ET AL: "Dicistronic targeting constructs: Reporters and modifiers of mammalian gene expression" PROCEEDINGS OF THE NATIONAL ACADEMY OF SCIENCES OF USA, vol. 91, no. 10, 10 May 1994, pages 4303-4307, XP000563920
- H. NIWA ET AL.: "Efficient selection for high-expression transfectants with a novel eukaryotic vector" GENE, vol. 108, 15 December 1991, pages 193-200, XP000569330 AMSTERDAM NL cited in the application
- TASHIRO K ET AL: "Signal sequence trap: A cloning strategy for secreted proteins and type I membrane proteins" SCIENCE, vol. 261, no. 5121, 30 July 1993, pages 600-603, XP002027998

## Description

This invention relates to methods of expressing DNA in cells, to vectors for expression of DNA in cells and to transfected cells. The invention also relates to assays carried out in transfected cells or differentiated derivatives of such cells. In particular the invention relates to transfection of and expression of DNA in non-human embryonic stem (ES) cells.

The wealth of sequence information now becoming available from the genome projects demands the development of new, high throughput systems for functional analysis. A powerful route to discovering and characterising genes involved in determination and differentiation in mammals is potentially available via the genetic manipulation of ES cells *in vitro*.

ES cells, which are derived from the pluripotential inner cell mass (ICM) of the preimplantation mouse embryo (2,3), retain the capacity for multilineage differentiation both *in vitro* (4,5) and *in vivo* (6,7). In principle, therefore, gene products which influence developmental decisions should be assayable in ES cell culture systems, whatever the source of the cells. However, there are major difficulties in analysing cDNA function by ES cell transfection. The frequency of isolating stable transfectants is low (<10⁻⁴ by electroporation, calcium phosphate co-precipitation or lipofection) and the great majority of transfectants show heterogeneous and unstable expression.

These problems are particularly significant in the case of cDNAs whose expression causes differentiation because differentiated ES cell progeny do not generally proliferate. In such cases transfectants may still be isolated but transgene expression will be minimal.

Episomal vectors have been used for functional screening in other cell types in order to increase the frequency of stable transfection and to achieve reliable transgene expression. However, previously described episomal vectors, for example based on Epstein-Barr virus (EBV) or bovine papilloma virus (BPV), have limitations both in host cell range and maintenance during long-term culture.

A modified extrachromosomal vector is known based on the replication system of murine polyoma virus (8). This plasmid, pMGD20neo, can be stably maintained as an episome in ES cells during long term culture. Importantly, the low levels of large T protein produced have no overt effect on the growth or differentiation properties of the ES cells (8,9). It is also known to maintain simultaneously with pMGD20neo a second episomal vector. Expression from the second vector was not possible hence pMGD20neo was used for cDNA expression. However, this vector already comprises two expression cassettes, one each for large T antigen and the neo selectable marker so its size constrains its use for expression of a third cassette containing a cDNA.

It is an object of the invention to provide a vector for transfection of and expression of DNA within a cell and a method of expressing DNA in a cell that overcomes or at least ameliorates the disadvantages identified in the art. An object of at least the preferred embodiments of the invention is to achieve, in a transfected cell, expression that is more stable and more homogenous than hitherto attainable. Further objects of preferred embodiments of the invention are to provide a method of expressing a DNA in a non-human embryonic cell in a more stable and more homogenous manner than hitherto attainable, and to provide for stable transfection of non-human embryonic cells at a higher frequency than can be obtained using conventional vectors.

The invention is based upon the maintenance of a vector within a cell, wherein maintenance of the vector is dependant upon the continued presence within the cell of a certain factor and wherein that factor is not expressed by the vector but is produced in or present in the cell in an amount sufficient to maintain the vector.

Accordingly the invention provides a method of expressing a DNA of interest in a non-human embryonic stem (ES), non-human embryonic carcinoma (EC) or non-human embryonic gonadal (EG) cell, comprising:
(a)
   (i) transfecting the cell with a first vector that expresses a replication factor; or
   (ii) otherwise obtaining a cell that expresses or will express the replication factor;
(b) transfecting the cell with a second vector, wherein
   (i) the second vector contains a DNA coding for a selectable marker in operative combination with a promoter for expression of the selectable marker;
   (ii) the second vector additionally contains a second DNA, being the DNA of interest, in operative combination with a promoter for expression of the DNA, and which does not code for a selectable marker;
   (iii) extrachromosomal replication of the second vector is dependent upon the presence within the cell of the replication factor; and
(c) expressing the second DNA.

As set out herein, the invention provides methods, vectors, uses of the vectors, cells and assays. The claimed vectors are for transfection of an ES, EC or EG cell. The claimed methods, uses of the vectors, cells and assays relate to non-human ES, EC and EG cells.

The replication factor is optionally non-toxic to the cell. Alternatively, the replication factor is toxic to the cell at high levels of expression but at low levels of expression is substantially non-toxic to the cell but at these low levels is present in sufficient amount to enable replication of the second vector.

Further, the replication factor preferably does not alter the ability of the cell to differentiate or proliferate, and may thus be regarded as being neutral to the cell phenotype. This enables the activities of the product of a cDNA to be investigated over a long time period and many cell generations without having to take account of possible interfering effects of the replication factor present within the cell. Again, the replication factor may be phenotype-neutral at all levels or may be neutral at a low level which is nevertheless a sufficient level to maintain the second vector within the cell.

The invention is of application to all cell types for which there exists, whether from a natural or synthetic source, a replication factor capable of maintaining in that cell type an episomal vector. The vector is preferably stably maintained, meaning it is maintained over a number of cell generations. The cell is preferably selected from the group consisting of non-human mammalian cells, in particular it is preferred that the cell is a non-human ES (embryonic stem) cell, a non-human EC (embryonic carcinoma) cell, or a non-human EG (embryonic gonadal) cell, or differentiated progeny of any such cell.

While reference is made to the second vector, it will be appreciated that the replication factor is optionally present in the cell other than following transfection with a first vector. For example a culture of cells that already express the replication factor may be obtainable from a third party.

In an embodiment of the invention described in detail below, the method comprises transfecting an ES cell with a first vector that expresses a viral replication factor, and thereafter transfecting the ES cell with a second vector that expresses a cDNA and is dependant upon presence of the viral replication factor for its extrachromosomal replication within the ES cell. The frequency of the first transfection step is generally low and may result in as few as 1 in 10⁵ successful stable transfectants - this level of success is recognised as typical in this art. However, the second transfection has surprisingly and advantageously found to result in a significantly higher frequency of successful stable transfectant colonies being obtained. The second transfection can be carried out with a 1% or higher success rate, which represents a 100-fold improvement over the art.

One suitable viral replication factor for mouse cells, in particular mouse ES cells, is polyoma large T antigen, in which case the cell of step (a) expresses the polyoma large T antigen and the second vector comprises an origin of replication that binds the polyoma large T antigen, such as the polyoma replication origin, referred to as *Ori*. Another suitable viral replication factor for primate cells is based upon Epstein Barr virus, in which the primate cell of step (a) expresses the EBNA-1 antigen and the second vector comprises an origin of replication that binds EBNA-1, such as *OriP*. Viral replication factors are generally species - specific and so expression of DNA according to the invention is dependent upon choice of a replication factor appropriate to the cell. Polyoma large T has been described for use in mouse cells. EβNA-1 is suitable for human cells. Still further systems are optionally based on papilloma virus replication factors, for human cells, or SV40 virus large T antigen, for simian cells, and further suitable replication factors may also be selected from functional variants, derivatives and analogues of these replication factors, such as temperature sensitive variants.

In use, the second vector is constructed according to standard techniques so as to contain a cDNA sequence or insert of interest operatively combined with a promoter to express the cDNA. The second vector is used to transfect an ES cell already expressing a replication factor and successful transfectants are recovered in which it is found that the second vector is stably maintained within the ES cell and expresses the cDNA with a more homogenous pattern than when prior art techniques are followed. Thus, the invention provides an advantageous method for expression of a cDNA in a cell.

In this context, "homogenous" in relation to expression of a cDNA in a colony of transfected ES cells is used to indicate that most cells, or a large proportion of cells, or preferably most cells, or more preferably substantially all cells, express the cDNA and "stable" is used to indicate that the cells continue to express the cDNA at a similar level and preferably at substantially the same level. In the examples carried out to date and described below, homogenous transfection is seen with the method of the invention to a greater extent than in the art methods. Also, in the examples carried out to date and described below the method results in more stable expression, meaning that expression does not alter over time. This has the advantage that study of the long term effects of a cDNA product is facilitated.

It is optional for the cell of step (a) first to be obtained or prepared by transfection of a cell by a first vector and for this then to be used for the starting cells for carrying out a plurality of separate transfections by second vectors containing different DNA inserts coding for different DNA products of interest. Following this procedure, the first transfection may be carried out with the level of success typically seen in conventional techniques and the ES cells obtained divided into separate colonies. The second transfections, introducing the DNA insert in the second vector, are then carried out with the higher levels of success typically seen in the methods of the invention.

In the case that the method comprises transfection with first and second vectors, it is preferable for the first vector to code for a selectable marker and for the second vector also to code for a selectable marker, though a different one. In a specific embodiment of the invention described below, the first vector codes for hygromycin resistance and the second codes for neomycin resistance. This allows selection of ES cells in which transfection by both first and second vectors has been successful.

It is a further embodiment of the invention for the method to comprise an additional transfection step with a third vector, wherein the third vector contains a cDNA, or is adapted to receive a cDNA, in operative combination with a promoter for expression of the cDNA, and extrachromosomal replication of the third vector is dependant upon presence within the ES cell of the replication factor. Transfection with the third vector is optionally at the same time as transfection with the second vector or subsequent thereto.

The second and third vectors preferably each comprise a selectable marker enabling selection of ES cells in which transfection has been successful. The respective selectable markers are preferably different if the method comprises transfection with both second and third vectors, and preferably different again from the selectable marker of the first vector.

It is a feature of particular embodiments of the invention that the second vector (and third or subsequent vectors if present) are not able to express the replication factor. In fact, in construction of the second vector from a vector comprising DNA encoding the replication factor it is preferable for that DNA to be largely or substantially completely deleted.

In a specific embodiment of the invention, the first vector is pMDG20neo and expresses polyoma large T antigen and the second vector comprises the natural target for polyoma large T antigen, namely *Ori*, expresses a cDNA of interest but does not express large T antigen. In use, the large T antigen is expressed by the first vector and binds to *Ori* of the second vector when it enters an ES cell, thus enabling replication of the second vector and its maintenance within the ES cell in an extrachromosomal state. In successful transfectants, the vector remains extrachromosomal, and this is believed to render the vector relatively immune from effects seen when a vector is integrated into the host ES cell genome, which effect may include silencing of the cDNA resulting in unstable and heterogeneous expression.

An alternative to use of the first episomal vector is to introduce into the cell a construct that expresses the replication factor and integrates with the cell genome. The construct should therefore include a DNA sequence coding for the replication factor and means for selection of cells in which the construct has successfully integrated; one example is a construct that comprises cDNA coding for, in order, large T antigen - an internal ribosome entry site (IRES) - Bgeo. A culture of cells is then obtained by selecting for cells that express the selectable marker, such as in this case by selection in G418. Staining with Xgal is used to identify transfectant clones which show stable and homogenous expression. The construct preferably comprises a promoter that gives stable, low level expression in transfected cells, such as the HMGCoA promoter for ES cells. The cells obtained can then be subjected to transfection with the second and optionally third and subsequent vectors.

In another embodiment of the invention the second vector comprises an inducible promoter. Some types of differentiated cells, derived from ES cells, can only be obtained with any reliability if a particular differentiating factor is expressed after a prior event. One example is neurone formation which generally only occurs after aggregation of cells. Thus, using an inducible promoter, expression of DNA that codes for the factor that leads to neurone formation can be controlled until the ES cells have suitably aggregated. Interferon responsive promoters are some examples of inducible promoters. Alternatively, the cDNA is designed to be in a non-functional form and to be capable of being modified into a functional form at a later time. One possibility is for the cDNA to be disrupted for example by termination sequences which are flanked by target sites for a site specific recombinase, such as loxP sites, removable by Cre recombinase, or frt sites removable by Flp recombinase. Cre and Flp can be fused to steroid hormone receptors in order to make their activity regulatable. After administration of steroid the Cre or Flp recombinase will translocate to the nucleus and there convert the cDNA into a functional form by excision of the disrupting sequence. It may also be desired to stop or inhibit or reduce replication of the second vector; the method optionally comprises using a site specific recombinase to present replication of the second vector. This can be achieved by deletion of a sequence in the vector to which the replication factor must bind in order for the vector to be replicated by the host cell.

The term DNA or cDNA is usually understood to refer to a DNA sequence that is transcribed into a mRNA that is translated into a polypeptide or protein. In the present invention the term is also intended to encompass any product of DNA expression. It thus includes DNA coding for an antisense RNA, or for an antisense ribozyme molecule.

The method of the invention is suitable for assaying effects of DNA expression, due to the stability and efficiency of expression achievable. Accordingly, the invention further relates to an assay for the effect of presence in a cell of any product of DNA expression - such as protein, polypeptide, antisense RNA, ribozyme RNA, transfer RNA or other. The method comprises steps (a) and (b) as described above wherein the second vector also contains a DNA coding for a selectable marker. The method further comprises selecting for cells that have been transfected with the second vector and maintaining the selected cells over a plurality of generations.

Step (a) may be carried out once and then steps (b) onwards repeated for different assays, and the method is of particular application to screening a cDNA library. Furthermore, two or more cDNAs can be expressed in the same cell to assay the effect of the combination of their respective expression products.

The invention also relates to a vector. Accordingly, the invention provides, in a second aspect, a vector for transfection of an ES, EC or EG cell so as to express a DNA of interest, wherein:
(i) the vector contains a DNA coding for a selectable marker in operative combination with a promoter for expression of the selectable marker;
(ii) the vector contains a second DNA, being the DNA of interest, in operative combination with a promoter for expression of the DNA, and which does not code for a selectable marker;
(iii) extrachromosomal replication of the vector is dependent upon presence within the cell of a replication factor; and
(iv) the vector does not express the replication factor.

The vector is characterized in preferred embodiments as described above in relation to the second vector of the first aspect of the invention.

It is an advantage of at least preferred embodiments of the invention that due to very high efficiency of stable secondary transfection (supertransfection) of cells, for example transfection of ES cells harbouring pMGD20neo with a second plasmid containing the polyoma replication origin (*Ori*) (8), that expression of DNA is stably and efficiently achieved from the second plasmid.

Another aspect of the present invention provides its use in a method of screening for new DNAs that encode signal sequences and proteins that are transported to the cell surface. The invention according provides a method of investigating the properties of a DNA sequence comprising expressing in a cell a composite DNA including (a) the DNA sequence under investigation, linked to (b) a DNA coding for a cell active protein, wherein
activity of the cell active protein is dependant upon transport of the cell active protein to the cell surface, and
the DNA of (b) does not code for a polypeptide capable of directing transportation of the cell active protein to the cell surface.

This offers the advantage that where the DNA of interest does indeed code for a sequence that transports a polypeptide to the cell surface, whether that polypeptide remains there or is ultimately secreted, this will be apparent from observation that the cell active protein has had or is having its known effect. Thus the method offers a convenient means of identifying DNA sequences that will transport proteins to the cell surface.

The method is suitably used for screening a library of DNAs to identify DNA sequences coding for signal polypeptide sequences that transport proteins to the cell surface. The cell active protein if transported to the cell surface may remain there or be secreted by the cell, and this distinction may be separately assayed, or example by examination of the make-up of the culture medium before and after the investigation.

One convenient way to obtain the DNA of (b) is by deleting or disabling, from a DNA encoding a cell surface or secreted protein, that portion of the DNA that codes for the polypeptide sequence responsible for transportation of the protein to the cell surface. The cell active protein is optionally a cell surface receptor and the DNA of (b) can thus encode a modified form of the receptor preprotein lacking a functional signal sequence. In a specific embodiment described below the IL-6 receptor is used as expression of the receptor in ES cells can be used to inhibit differentiation of the cells - a readily observable property of the cell active protein. Gross morphological or proliferative changes induced in the cell by the cell active protein are of course readily observed, though the invention is of application to any cell active protein whose activity, when it is transported to the cell surface and / or secreted, can be assayed.

A specific embodiment of this aspect of the invention comprises expressing the composite DNA by:
(a)
   (i) transfecting a cell with a first vector that expresses a replication factor; or
   (ii) otherwise obtaining a cell that expresses the replication factor;
(b) transfecting the cell with a second vector, wherein
   (i) the second vector contains the composite DNA in operative combination with a promoter for expression of the composite DNA;
   (ii) the second vector also contains a DNA coding for a selectable marker in operative combination with a promoter for expression of the selectable marker; and
   (iii) extrachromosomal replication of the second vector is dependant upon presence within the cell of the replication factor;
(c) selecting for cells that have been transfected with the second vector; and
(d) maintaining the selected cells over a plurality of generations so as to assay the effect of expression of the composite DNA.

If many investigations are to be carried out it is preferred that step (a) is carried out once and the cells obtained are divided and used for a plurality of separate methods in which steps (b)-(d) are carried out a plurality of times with second vectors containing different DNA sequences. This offers the advantage that typically the first transfection step is of lower efficiency than the second, so the method avoids having to repeat the low efficiency step too often.

It is particularly preferred that the method is used for identification of a DNA coding for a cell surface or secreted protein, and using the method to screen a library of DNAs provides a means of carrying out the screen for discovery of such DNAs and investigation of their properties. More especially, the method is for discovery of hitherto unknown or uncharacterized cell surface or secreted proteins, or for location of the coding sequence of known proteins of this type.

This aspect of the invention optionally further incorporates in preferred embodiments features of transfection of cells described above in relation to other aspects of the present invention.

The invention enables development of a series of vectors which give highly efficient and robust expression of transgenes in cells. Cloned cDNAs of interest can rapidly be characterised using this system. It is also applicable to the discovery of novel regulatory molecules through functional expression screening of cDNA libraries.

Due to their pluripotent and proliferative character, key cellular processes such as viability, propagation, determination and differentiation, can be analyzed in transfected ES cells. The "supertransfection" system of the invention overcomes the limitations associated with conventional cDNA transfection and opens a powerful new route to gene discovery and characterisation in mammals.

Key features of the episomal supertransfection system, described according to the examples below, are that very high efficiencies of stable transfection are obtained and that cDNA expression is homogeneous, stable and reliably dictated by promoter strength. The increased efficiency of isolating stable transfectants is significant because it allows reliable detection of cDNAs whose expression results in cell death or differentiation. In addition a high transfection efficiency is generally advantageous for any high throughput assay system and is essential for functional cDNA library screening. The reliability of cDNA expression is critical for functional studies and the robust nature of expression from episomal vectors contrasts favourably with the variable and unstable expression observed in conventional ES cell transfectants.

Heterogeneous expression of integrated transgenes is not an artefact arising from use of bacterial *lacZ* as a reporter gene, firstly because similar observations have been made using mammalian thy-1 as a reporter in F9 cells, and secondly because ubiquitous expression of *lacZ* can readily be obtained following gene trap integrations (23,24). The expression pattern throughout the population cannot be determined by Northern blot but can only be revealed by *in situ* hybridization or use of a linked reporter gene such as IRES-*lacZ* (25) Heterogeneous expression, which previously occurred in the great majority of transfected clones following stable integration, gave unclear or misleading results on the phenotypic consequences of transgene expression.

The difference in expression pattern between conventional transfectants and episomal supertransfectants of the invention arises because an extrachromosomal copy of a transgene is not subject to alteration during the integration process nor to modification arising from the genomic sequences flanking an integration site. The so-called "position effect" can modify both the level and pattern of transgene expression in stable transfectants. Furthermore, the expression of integrated transgenes is often suppressed over several generations in ES cell cultures. This silencing phenomenon contributes to the high backgrounds which can be obtained in double replacement type targeting strategies (26). It has been observed in stable transfectants with different transgenes driven by viral promoters or minimal mammalian promoters such as the widely used human β-actin and mouse PGK-1 promoter elements. One hypothesis to explain this phenomenon is that transgenes may become targets of de novo methyltransferase in stem cells (27). Macleod et al. (28) reported that a methylation free locus could be generated in transgenic mice by introduction of the whole CpG island of the aprt promoter.

Whatever the molecular mechanism of silencing, it appears not to occur to episomally maintained transgenes in vectors of the invention. In addition, the level of expression obtained from vectors of the invention is reliably dictated by promoter strength and can predictably be varied over at least a 10-fold range by appropriate choice of promoter. Episomal constructs of the invention thus offer considerable advantages for functional expression studies in ES cells.

Functional cDNA expression cloning is a powerful method for direct isolation of important genes. The expression screening approach has often been employed to isolate cDNAs encoding surface and secreted molecules via transient expression, for example in COS cells. In a few cases EBV-based systems have also been applied to isolate intracellular regulatory genes via stable expression in the target cells (29-32). The high efficiency of supertransfection in the polyoma system of the invention indicates that this approach could be applied to functional cloning in ES cells. Based on a transfection efficiency of 2.5%, a library of 5x10⁵ cDNA clones could be screened by electroporation of 2x10⁷ cells with 100µg DNA. For an effective library screen, the majority of transfectants should only take up a single plasmid. It is also advantageous if the cDNAs can readily be recovered in unrearranged form. Both of these conditions are satisfied by the episomal supertransfection system. By screening libraries prepared from undifferentiated ES cells it may be possible to isolate cDNAs whose products mediate self-renewal. In this case direct selection can be applied for colony formation in the absence of LIF. For cDNAs whose products direct differentiation, however, it will be necessary either to screen pools through several rounds or to incorporate an inducible promoter into the episome.

Recently, several improved protocols for *in vitro* differentiation of ES cells have been reported, which promote efficient generation of, for example, haematopoietic cells (33), neurons (34) or cardiomyocytes (35). The episomal expression strategy of the invention can be applied for gain-of-function assays and screens during these differentiation programmes. It can also be used for loss-of-function analyses via overexpression of anti-sense RNA or dominant-negative mutants. Combination of these differentiation systems with the episomal expression system will therefore provide powerful tools for analysing cell determination and differentiation events.

The invention is now described with reference to the accompanying drawings in which:
Fig. 1 shows the structure of the episomal expression vector pHPCAG;
Fig. 2 shows supertransfection efficiency of pHPCAG in MG1.19 ES cells;
Fig. 3 shows DNA hybridisation analysis of Hirt supernatants from supertransfectants;
Fig. 4 shows the effect of vector size on supertransfection efficiency;
Fig. 5 shows expression of β-galactosidase in MG1.19 transfectants;
Fig. 6 shows the restriction pattern of plasmid DNAs recovered from pHPCAG-*lacZ* supertransfectant clone;
Fig. 7 shows induction of differentiation by expression of STAT3F in MG 1.19 ES cells;
Fig. 8 shows co-supertransfection of STAT3F with wild type STAT expression vectors;
Fig. 9 shows linker sequences for use in an assay of the invention;

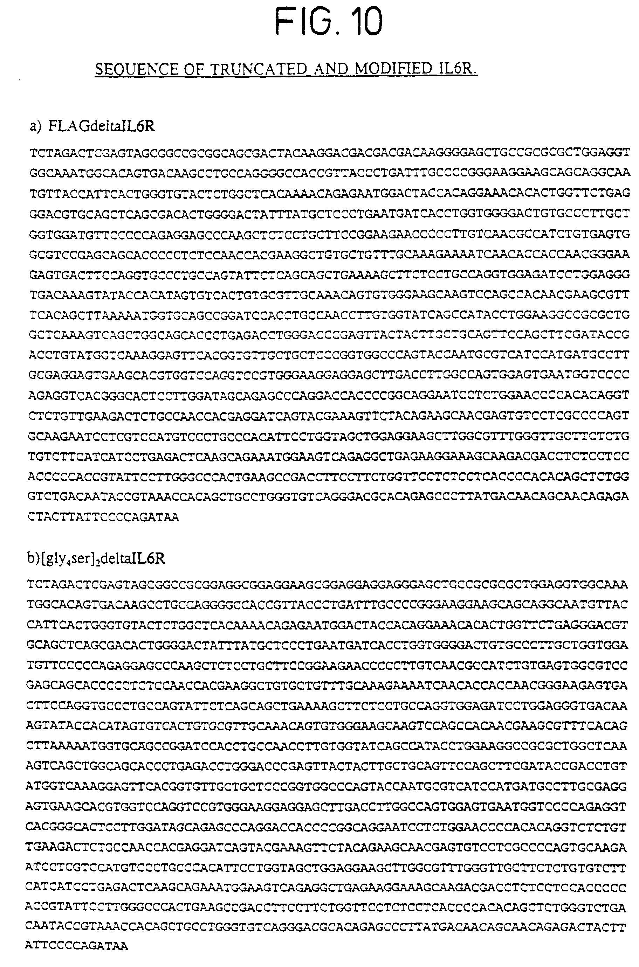

Fig. 10 shows DNA sequences coding for truncated and modified IL6R; and
Fig. 11 shows a vector for use in an assay of the invention.

In more detail:

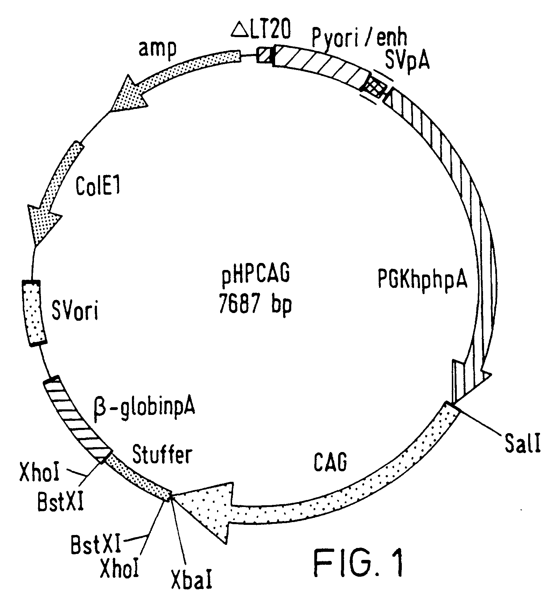

Figure 1 shows the structure of the episomal expression vector pHPCAG. cDNAs can be introduced between two *Bst*XI sites using *Bst*XI adaptors. Abbreviations: ΔLT20: deleted polyoma large T expression cassette LT20; Pyori/enh: mouse polyoma virus replication origin and mouse polyoma mutant enhancer derived from F101 strain; SVpA: SV40 polyA addition signal; PGK*hph*pA: hygromycin B phosphotransferase gene expression cassette with mouse phosphoglycerokinase-1 (PGK) promoter and polyA addition signal; CAG: combined CAG expression unit; β-globinpA: rabbit β-globin polyA addition signal; SVori: SV40 replication origin; ColE1ori: ColE1 replication origin; *amp: E.coli* β-lactamase gene conferring resistance to ampicillin.

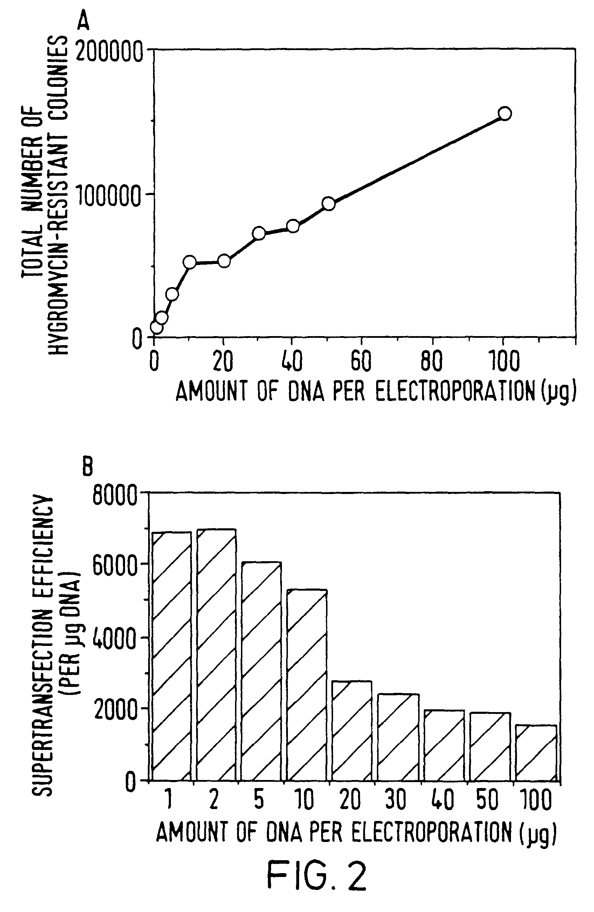

Figure 2 shows supertransfection efficiency of pHPCAG in MG1.19 ES cells.
   (A) shows numbers of transfectant colonies per microgram of pHPCAG DNA. 5x10⁶ MG1.19 ES cells were supertransfected with the indicated amounts of supercoiled pHPCAG followed by selection with hygromycin B for 8 days. The resulting number of drug-resistant colonies were scored and efficiency per µg DNA calculated.
   (B) shows total numbers of transfectant colonies plotted against total amount of plasmid DNA.

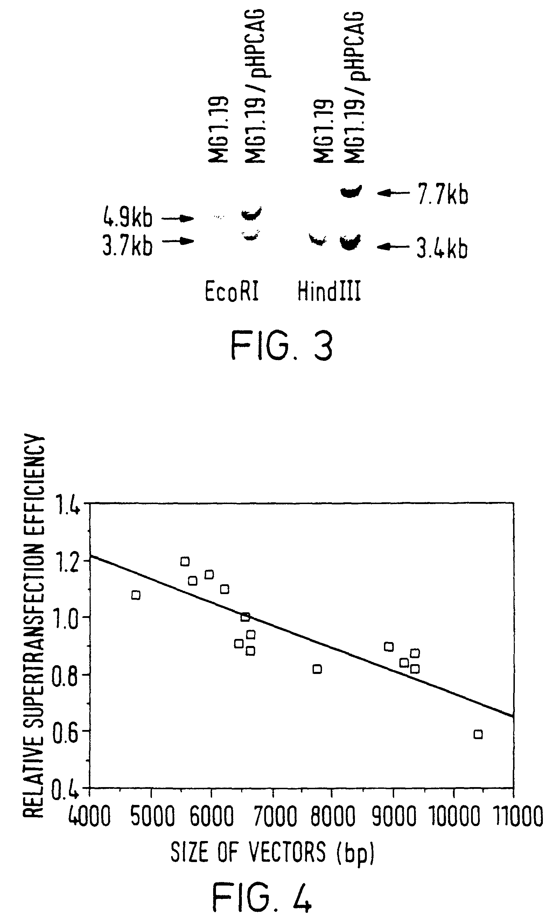

Figure 3 shows DNA hybridisation analysis of Hirt supernatants from supertransfectants. Hirt supernatants were prepared from 5x10⁶ parental MG1.19 cells and pooled pHPCAG supertransfectants. 1/20 of each sample was digested with either Eco RI or *Hind*III and analyzed by filter hybridisation using a 344bp *Sca* I-Sspl fragment from pUC19 which is common to both pMGD20*neo* and pHPCAG.
Figure 4 shows the effect of vector size on supertransfection efficiency. 20µg of each of the supercoiled vectors pLT20Δ*Nde*l*hph* (4.7), pLT20Δ*BstX*l*hph* (5.5), pLT20Δ*AlwN*l*hph* (5.6), pLT20Δ*Sac*l*hph* (5.9), ptkp (6.2), pSV40e/p (6.4), PGK*hph*ΔLT20 (6.5), pmPGKp (6.6), phBAp (6.6), pHPCAG (7.7), ptkp-*lacZ* (8.9), pSV40e/p-*lacZ* (9.1), pmPGKp-*lacZ* (9.3), phBAp-*lacZ* (9.3), and pHPCAG-*lacZ* (10.4) were individually supertransfected into 5x10⁶ MG1.19 ES cells. The resulting numbers of hygromycin B resistant colonies were scored after 8 days. Transfection efficiencies are normalised relative PGKhphΔLT20.

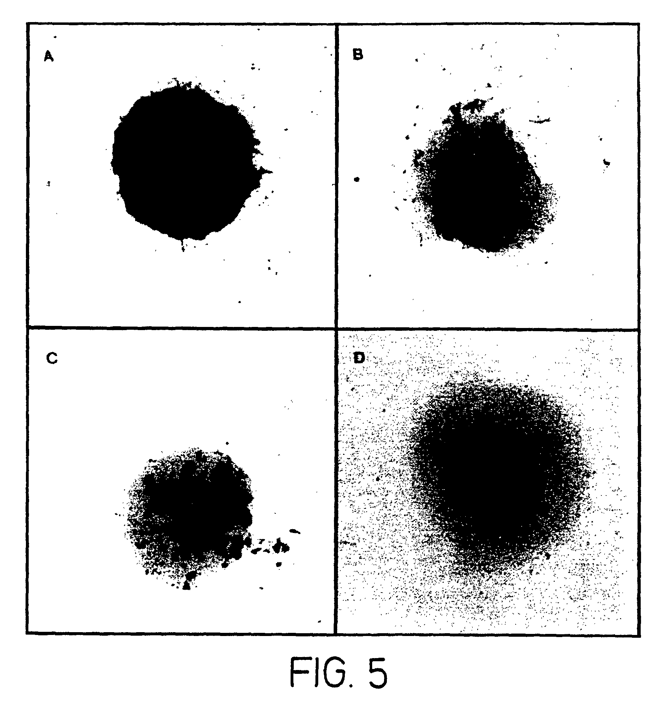

Figure 5 shows expression of β-galactosidase in MG1.19 transfectants. Primary colonies were stained with Xgal after 8 days of selection.
   (A) shows typical homogeneous staining pattern obtained following supertransfection with supercoiled pHPCAG-*lacZ*.
   (B)shows heterogeneous staining pattern obtained in minority of clones following supertransfection with supercoiled pHPCAG-*lacZ*.
   (C) shows heterogeneous staining pattern typically observed following electroporation of linearized pHPCAG-*lacZ* and stable integration.
   (D) shows rare faint staining pattern obtained after supertransfection with supercoiled pHPCAG-*lacZ*.

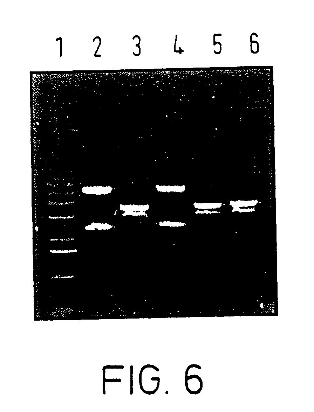

Figure 6 shows the restriction pattern of plasmid DNAs recovered from pHPCAG-*lacZ* supertransfectant clone.
   A supertransfectant MG1.19 clone carrying pHPCAG-*lacZ* was cultured for 60 days in the presence of hygromycin B. Hirt DNA was then prepared and electrotransformed into *E.coli* DH10B cells. Plasmid DNAs were recovered from transformants, digested with *EcoR*I, resolved by electrophoresis on 1.0% agarose gel and visualised by ethidium bromide staining. Expected fragment sizes: pMGD20neo, 4852bp and 2884bp; pHHPCAG-*lacZ*, 3697bp, 2810bp, 783bp and 397bp. Lane 1: size marker (1kb ladder:BRL); lane 2: control pMGD20; lane 3 : control pHPCAG-*lacZ*; lane 4: recovered pMGD20; lane 5,6: recovered pHPCAG-*lacZ*.

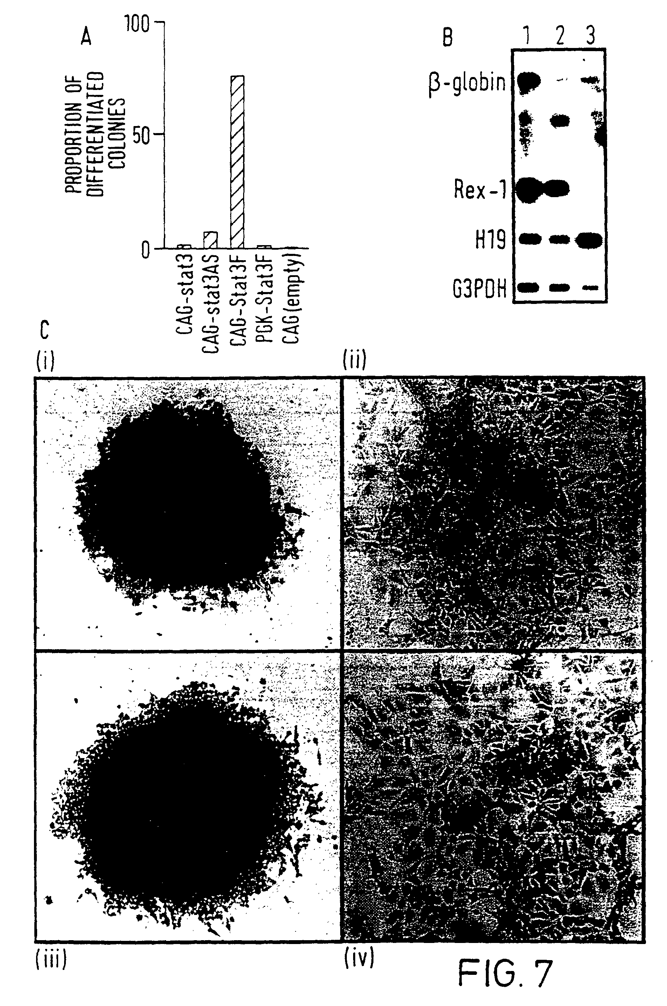

Figure 7 shows induction of differentiation by expression of STAT3F in MG 1.19 ES cells.
   (A)shows proportion of differentiated colonies in LIF-supplemented medium resulting from supertransfection of STAT3, antisense STAT3 and STAT3F expression vectors. Colonies were fixed and stained with Leishman's reagent after 8 days selection and numbers of stem cell colonies and differentiated colonies scored.
   (B) shows marker gene expression in STAT3F supertransfectants: Expression of marker genes in pools of MG 1.19 cells supertransfected with STAT3 (lane 1), STAT3 antisense (lane 2) and STAT3F (lane 3) expression vectors. Total RNA was prepared after 8 days of selection in LIF-supplemented medium and 5µg aliquots analyzed by filter hybridisation with β-globin, Rex-1, H19 and G3PDH probes. The β-globin probe detects all transgene mRNA species generated from pHPCAG, including an alternatively spliced product from the antisense construct.
   (C)shows photomicrographs of representative colonies 8 days after supertransfection with (i) STAT3, (ii) STAT3F, and (iii) empty expression vectors and selection in the presence of LIF, or, (iv) induction of differentiation by culture in the absence of LIF for 8 days.

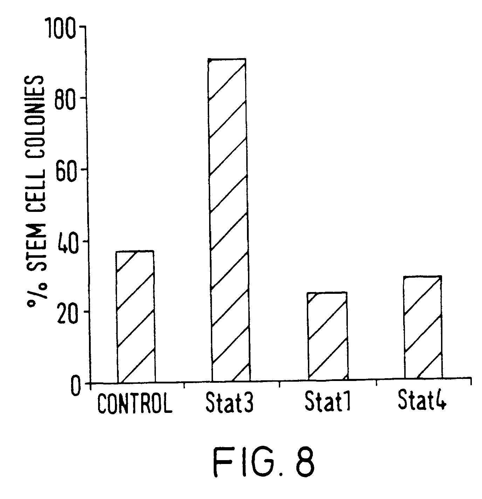

Figure 8 shows co-supertransfection of STAT3F with wild type STAT expression vectors. Proportions of undifferentiated stem cell colonies generated after co-supertransfection of MG1.19 ES cells with 10µg pBPCAGGS-STAT3F plus 10µg pHPCAG vector containing stuffer (control), STAT3, STAT1 or STAT4 inserts. After 8 days selection with 80µg/ml of hygromycin B plus 20µg/ml of blasticidin S, colonies were fixed and stained with Leishman's reagent.

### EXAMPLE 1

### Materials and Methods

### Vector constructions.

Standard recombinant DNA methods were used to construct all plasmids(10) . Plasmid pHPCAG (Fig 1) was constructed from pMGD20neo(8). The PGKneopolyA sequence was replaced by a hygromycin resistance marker, PGKhphpA, and large T sequences were deleted (see Results). A *Sal*I-*Sca*I fragment containing the CAG expression unit, a *Bst*XI stuffer sequence, the polyA addition signal derived from the rabbit β-globin gene and an SV40 replication origin (11) was inserted. Coding sequences for β-galactosidase, LIF or interleukin-2 were introduced between the BstXI sites.

For construction of episomal expression vectors with alternative promoters, the *Sal*l-Xbal fragment containing the CAG expression unit in pHPCAG-*lacZ* was replaced with the 344 bp SV40 enhancer/promoter (SV40e/p), the 466 bp human β-actin promoter (hBA), the 502 bp mouse phosphoglycerate kinase promoter (mPGK) and the 90 bp HSV-tk minimal promoter (tk), resulting in pHPSV40e/p-*lacZ*, pHPhBA-*lacZ*, pHPmPGK-*lacZ* and pHPtk-*lacZ*, respectively.

Episomal vectors with alternative selection markers were constructed by replacing the PGKhphpA cassette in pHPCAG with the SV*bsr*pA cassette carrying the *E.coli* blasticidin S deaminase (*bsr*) gene derived from pSV2bsr (Waken Seiyaku) or the hCMVzeopA cassette carrying the *Streptoalloteichus* bleomycin resistant gene (*Sh ble*) derived from pZeoSV (Invitrogen) to generate pBPCAGGS and pZPCAGGS, respectively.

### Cell culture and transfection.

MG1.19 ES cells are derivatives of the CCE line which stably maintain around 20 episomal copies of pMGDneo(8). They were maintained on gelatin-coated plates in Glasgow modified Eagle's medium (GMEM, Gibco-BRL) supplemented with 10% fetal calf serum, 0.1 mM β-mercaptoethanol, non-essential amino acids, 200 µg/ml G418, and 100U/ml LIF produced in COS-7 cells(11,12). For supertransfection, routinely, 5x10⁶ MG1.19 cells were suspended in 800 µl of PBS, incubated with 20 µg of supercoiled vector DNA for 10 min on ice, and electroporated at 200V/960µF using a Bio-Rad gene pulser. Cells were transferred into gelatinized plates and allowed to recover overnight before addition of appropriate selection agent. Histochemical staining for β-galactosidase was carried out with 5-bromo-4-chloro-3-indolyl β-D-galactopyranoside (X-gal) (13), and β-galactosidase activity was measured by incubation of cell extracts with o-nitrophenyl-β-D-galactopyranoside (ONPG). Differentiation was induced in monolayer culture as described (12).

### Analysis of episomal vectors in the supertransfectants.

Hirt supernatants were prepared as described (14). For amplification of recovered episomal vectors, electrocompetent *E. coli* DH10B cells were transformed by electroporation at 2500V/25µF/200½.

### Results

### Construction of an episomal expression vector.

Polyoma-based plasmids have recently been reported to be competent for episomal propagation in ES cells (8). The plasmid pMGD20neo contains a modified large T expression unit called LT20, the viral origin of replication (*Ori*), and the PGKneopA cassette as a selectable marker. This plasmid can be maintained as an extrachromosomal element in wild-type ES cells. It can be modified to include a cDNA expression unit (9). However, the low frequency of conventional stable transfection of ES cells (Å 1 x 10⁻⁵) remains a limiting feature. Furthermore, episomal propagation only occurs in 10-15% of primary transfectants (8,9) .

A second plasmid has been described which can be maintained as an episome only in ES cells which independently express the large T protein (8). This plasmid, PGKhphΔLT20, contains LT20 with a large deletion in its coding sequence, *Ori*, and PGK*hph*pA as a selectable marker. When introduced into a cell line such as MG1.19, in which episomal maintenance of pMGDneo has already been established, the yield of hygromycin B resistant stable transfectants is extremely high. This phenomenon of supertransfection is presumed to arise from the pre-existence of large T protein in the recipient cells.

In the studies reported below the modification and use of supertransfection vectors for cDNA expression is characterised.

### Size of vector

PGK*hph*ΔLT20 retains part of the large T coding sequence. We made a series of deletions in the ΔLT20 sequence to minimize the vector size and thereby increase the capacity for inserts and reduce potential bias in the construction and screening of cDNA libraries. The supertransfection efficiency of four derivative plasmids was then compared in MG1.19 cells. All showed comparable supertransfection efficiency to PGK*hph*ΔLT20 (data not shown). The smallest, pLT20Δ*Nde*I*hph*, has a deletion of 2953 bp, yielding an episomal vector backbone of only 4.7kb.

### Expression unit

Into this minimal episomal vector we introduced a cDNA expression unit. Transcriptional initiation signals are supplied by the CAG cassette (11), which comprises the human cytomegalovirus immediate early enhancer, a 1kb fragment of the chicken β-actin gene (promoter, non-coding first exon and first intron), and a splice acceptor derived from the rabbit β-globin gene. This combination has been shown to direct strong expression of cDNAs in undifferentiated stem cells. The resulting expression vector, pHPCAG (Fig 1), contains the CAG sequences followed by the *Bst*XI stuffer sequence derived from pCDM8 as a cDNA cloning site, and a polyA addition signal derived from the rabbit β-globin gene. In addition the plasmid contains the PGKhphpA (15) cassette for hygromycin selection of ES cell transfectants, the polyoma*Ori* with pyF101-derived mutant enhancer element (16) for stable episomal replication in cells expressing polyoma large T protein, and the β-lactamase (amp) gene and prokaryotic replication origin for amplification in *E. coli*. The SV40 *Ori* is also present to allow for transient episomal replication in mammalian host cells expressing SV40 largeT, such as COS cells (17) .

### Characterization of supertransfection.

The parameters of supertransfection with pHPCAG and derivatives were investigated. First, 5x10⁶ MG1.19 cells were electroporated with various amount of supercoiled pHPCAG, selected in medium containing 80 µg/ml of hygromycin B for 8 days, and the number of stem cell colonies scored after Leishman's staining(12). Although the highest efficiency per µg DNA was observed with minimum amounts (1-2 µg) of vector DNA (Fig. 2B), the total yield of hygromycin B resistant colonies increased with increasing amount of plasmid (Fig 2A). Saturation was not reached over the range of plasmid concentrations tested. With 100 µg plasmid DNA, 150,000 hygromycin B-resistant colonies were obtained, representing 3% of total treated cells. Disablement for episomal replication by linearisation of pHPCAG prior to electroporation reduced this transfection efficiency to less than 0.01%.

Next, increasing numbers of MG 1.19 cells were subjected to electroporation with 100 µg of pHPCAG DNA. Comparable stable transfection efficiencies in the range 3-6% were obtained with up to 2.5x10⁷ cells.

The copy number of pHPCAG in the supertransfectants was analyzed by preparation of Hirt supernatants followed by filter hybridisation. This analysis revealed that supertransfected cells carried approximately 20 copies each of pMGDneo and pHPCAG (Fig. 3).

These data demonstrate that the efficiency of supertransfection with pHPCAG is extremely high. However, episomal vectors can be limited in their capacity for inserts because increased size may cause inefficient replication or instability. To investigate this issue in the ES cell system, episomal vectors of different size were supertransfected into MG 1.19 cells. The numbers of supertransfectant colonies were scored and plotted against vector size (Fig. 4). These data indicate that there is a progressive reduction in transfection efficiency with increasing plasmid size. In particular, the largest plasmid tested, a derivative of pHPCAG with a 3kb *lacZ* insert (total size 10.4kb) showed a 50% reduction in colony number. However, that this may not be due entirely to the size of the plasmid because the very high levels of β-galactosidase expression may exert some toxic effects (see below).

### lacZ expression in supertransfectants.

To evaluate the level and pattern of expression of transgenes from pHPCAG, the *E.coli* β-galactosidase (*lacZ*) gene was introduced into this vector. The resulting vector, pHPCAG-*lacZ*, was introduced into MG1.19 cells and supertransfectants isolated by selection with 80 µg/ml of hygromycin B for 8 days. The number of colonies isolated was 50% of the number obtained in a parallel supertransfection with pHPCAG (see above). The colonies were smaller and many of the cells showed an abnormal spindle-shaped morphology. These effects were not observed with several other inserts in pHPCAG and are suggestive of a toxic effect of the high level *lacZ* expression. The primary supertransfectants were stained with X-gal and the staining pattern examined under phase-contrast microscopy. Staining was detectable after 5 minutes incubation and was intense by 1 hour. This level of β-galactosidase activity is significantly higher than we have observed from a variety of integrated expression constructs.

Approximately 80% of supertransfectant colonies showed ubiquitous expression (>90% cell positive) as shown in Fig.5-A (i). Of the remainder, 15% showed heterogeneous expression (Fig.5-A (ii)), and 5% showed little or no staining (Fig.5-A (iv)). The latter two classes are likely to arise as a result of vector integration which occurs in up to 20% of supertransfectants (8). In transfectants derived by electroporation of linearized pHPCAG-*lacZ* into MG1.19 cells (which results in vector integration in the majority of clones), only 15 % of colonies showed homogeneous staining whereas 70% of colonies stained heterogeneously (Fig.5-A (iii)), and 15% showed no expression.

Analysis of expanded clones from each class of transfectant established that this difference in expression characteristics was stable. Twelve of 13 expanded supertransfectants expressed *lacZ* homogeneously. In contrast, only 4 out of 24 clones derived using linearized vector showed homogeneous expression. This is consistent with our previous observations on integrated expression constructs in ES cells. In fact the CAG unit gives a significantly higher frequency of colonies which show stable ubiquitous expression than other promoters we have examined.

The difference in staining pattern between episomally maintained and integrated vectors indicates that the former escape modifying influences arising from integration and reliably give full activity of the expression unit.

### Comparison of expression with various promoters on episomal vector.

An ability reliably to generate predetermined levels of expression would be a important attribute for a transgene expression system. The previous observations suggested that episomal vectors offered potential to achieve unmodified expression. Various promoters with different strengths in undifferentiated stem cells were therefore introduced into the episomal vector by replacing the CAG expression unit of pHPCAG-*lacZ*. Expression of the *lacZ* reporter was then assayed in both transient and stable supertransfectants (Table 1). The relative ratio of β-galactosidase activity obtained from the SV40 enhancer/promoter complex, the human β-actin promoter, the mouse PGK-1 promoter and the HSV-tk minimal promoter in transient transfectant was retained in stable supertransfectants. The CAG expression unit showed strongest activity in the tested constructs in both transient and stable transfectants. In this case, however, the relative ratio in transient transfectants, 19 times higher than SV40, was significantly reduced in stable transfectants. This may arise from an elimination of strong expressants due to a toxic effect of high *lacZ* expression (see above). A reduced number of supertransfectants and smaller size of colonies was observed only with the CAG vector.

### Stability of supertransfected episomal expression vector during long-term culture and differentiation of host cells.

A critical limitation of previously described episomal vectors is their instability during long-term culture. Many episomal vectors undergo integration into the host genome after long-term culture, resulting in a reduction in expression and inability to recover transgenes by preparing Hirt supernatants. To test the stability of the supertransfection system, four pHPCAG-*lacZ* supertransfectant clones were cultured for 60 days (approximately 90 generations) under continuous selection with 80 µg/ml of hygromycin B. Three of the four clones maintained relatively constant levels of β-galactosidase activity determined by ONPG assay and uniform expression as revealed by Xgal staining. The fourth clone showed unstable and variegated expression, as commonly observed on vector integration. Hirt supernatants were prepared from one of the stably expressing clones at the end of the 60 day culture period. Filter hybridization analysis of the Hirt DNA indicated that the ES cells carried approximately 20 copies of pMGD20 and 5 copies of pHPCAG-*lacZ* per cell (data not shown). The lower copy number of pHPCAG-*lacZ* may be due to its larger size and/or the toxic effect of strong *lacZ* expression. The Hirt DNA was transformed into *E.coli* for further analysis. Of the bacterial transformants, 20% carried pHPCAG-*lacZ* and the remainder carried pMGDneo20, in good agreement with the hybridization data. Restriction mapping showed no evidence of rearrangement in either plasmid (Figure 6).

In the experiment above, cells were maintained under selection with hygromycin B. In the absence of selection pressure, supertransfectant clones lost expression of β-galactosidase over several passages in culture. This might indicate an intrinsic instability of supertransfected episomal vectors. However, it could also reflect a selective disadvantage for ES cells which express high levels of β-galactosidase. It is noteworthy in this regard that the primary episome, pMGD20neo, is stable in the absence of selection(8) .

Stability of expression from pHPCAG-*lacZ* during the *in vitro* differentiation of ES cells was also analyzed. Differentiation was induced in three ways: withdrawal of LIF; exposure to retinoic acid; and treatment with 3-methoxybenzamide(18). After 6 days the differentiated progeny stained ubiquitously in all three cases (data not shown).

These data indicate that supertransfected episomal vectors can be maintained in an extrachromosomal state and direct strong expression of transgenes during long-term self-renewal and differentiation *in vitro*.

### Production and secretion of the cytokine LIF from an episomal ES cell expression vector.

The pHPCAG-*lacZ* plasmid can efficiently direct strong and homogeneous expression of the cytoplasmic *lacZ* reporter gene. We next investigated expression of a secreted molecule, the cytokine LIF. LIF is an essential supplement to ES cell culture medium because it inhibits differentiation of the stem cells (19,20). Expression of LIF can readily be assayed by formation of stem cell colonies in media lacking the cytokine.

Episomal vectors for expression of another cytokine, interleukin-2 (which has no effect on ES cell phenotype), and for LIF were electroporated in parallel into MG1.19 cells. The cells were seeded at low density (1.5x10⁴ and 5x10³ cells per 90mm plate) to avoid the rescue effect which arises from the production of LIF by differentiated ES cell progeny (21), and cultured with 80 µg/ml of hygromycin B for 8 days. pHPCAG-*il2* generated large numbers of stem cell colonies in medium supplemented with LIF, but none in the absence of LIF. pHPCAG-*lif* in contrast produced comparable numbers of healthy stem cell colonies in both the presence and absence of exogenous LIF (Table 2). These colonies could be expanded and propagated without LIF-supplementation of the medium. These data confirm previous observations that increased autocrine expression of LIF renders ES cells factor-independent (22) and establish that secreted proteins are produced efficiently and stably by this episomal expression system.

### Co-supertransfection of episomal vectors.

Introduction of two or more different transgenes into cells is often required for analysis of protein interactions and/or co-operative function. The poor efficiency of homogeneous expression in conventional transfectants is a major obstacle for such investigations in ES cells. To test the possibility that the episomal approach could be applied to co-express multiple cDNAs, we constructed episomal expression vectors with different selection markers. Co-supertransfection of episomal vectors was then assessed.

The basic episomal expression vector pHPCAG carries the hygromycin phosphotransferase gene driven by mouse PGK-1 promoter (PGKh*php*A). We prepared episomal vectors which carry the zeocin-resistance gene driven by the human cytomegalovirus immediate-early promoter (pZPCAG), or the blasticidin S-resistance gene driven by the SV40 enhancer/promoter (pBPCAG) by substitution of the PGK*hph*pA cassette in pHPCAG. These vectors were supertransfected into MG1.19 cells followed by 8 days selection with the appropriate antibiotic. Comparison of the numbers of resulting drug-resistant colonies (Table 3) revealed that these selection systems are slightly less efficient than hygromycin B selection but nonetheless enable large numbers of supertransfectants to be isolated.

ES cells harbouring two different episomal vectors can be isolated by repeated supertransfection. Supertransfectants carrying pHPCAG can be transfected again with pBPCAG or pZPCAG, with comparable efficiency to the original supertransfection into MG1.19 ES cells (data not shown). This should allow establishment of efficient screens for assaying functional interactions between gene products.

The effects of co-electoporation of supertransfection vectors were also investigated. pHPCAG (10 µg) and pBPCAG (10 µg) were co-electroporated into 5x10⁶ MG1.19 cells. Cells were selected in hygromycin B or blasticidin S only, or both, for 8 days and the number of drug-resistant colonies scored in each case. The numbers of hygromycin or blasticidin S single-resistant colonies were 39,000 and 13,000, respectively, while the number of double-resistant colonies was 1,200. Thus the apparent efficiency of incorporation of both plasmids was less than 10%. Similar results were obtained on co-supertransfection of pHPCAG and pZPCAG (not shown). These data suggest that the majority of supertransfectants incorporate only one plasmid under these electroporation conditions. This is significant for application of the episomal system to functional cDNA library screening.

### EXAMPLE 2

The effects of overexpression of a large number of transgenes in ES cells were investigated by construction of vectors based on pHPCAG and including a DNA insert coding for the transgene being investigated. 5 x 10⁶ ES MG1.19 cells were supertransfected with 20 µg of expression vectors and selected with 80 µg/ml of hygromycin B for 8 days. The numbers of drug-resistant colonies were counted and normalised relative to numbers obtained with empty vector. The results are shown in Table 4.

### EXAMPLE 3

### Inhibition of STAT3 activation blocks self-renewal and promotes differentiation

To assess directly the requirement for STAT3 activation in ES cell self-renewal, we exploited a dominant interfering mutant form of STAT3, STAT3F. In this mutant (Minami *et al*., 1996), the tyrosine residue at amino acid position 705 is mutated to phenylalanine. Phosphorylation of Tyr705 is required for dimerization and nuclear translocation. When expressed at high level, STAT3F has been shown to block the activation of endogenous STAT3 in various cell types, possibly by titrating out receptor docking sites (Fukada *et al*., 1996; Minami *et al*., 1996; Nakajima *et al*., 1996; Bonni *et al*., 1997; Ihara *et al*., 1997).

Using conventional transfection approaches we were unable to recover ES cell transfectants showing stable high level expression of STAT3F. In parallel experiments, however, transfection of the LIF-independent embryonal carcinoma cell line P19 yielded multiple expressing clones. This suggested that blockade of STAT3 activation in ES cells specifically resulted in cell death, growth arrest or differentiation. The transfection and expression strategy of the invention was therefore adopted to enable characterisation of the consequences of STAT3F expression.

The STAT3F mutant cDNA was introduced into the supertransfection vector pHPCAG. The wild type STAT3 coding sequence was also introduced, in both sense and antisense orientations. The three constructs were electroporated into MG1.19 cells which harbour a large T expression plasmid and can be supertransfected with constructs containing the polyoma origin (Gassmann *et al*., 1995). Supertransfectants were isolated by selection in hygromycin B for 8 days in the presence of LIF. Colonies were fixed, stained with Leishman's reagent, counted, and scored for the presence of stem cells and differentiated cells. More than 95% of colonies obtained following supertransfection with control or wild type STAT3 vector were stem cell colonies (Figure 7A). A modest increase in the proportion of differentiated colonies was obtained with the antisense construct. The STAT3F vector, however, yielded predominantly differentiated colonies. A decrease in total number of colonies was also observed after supertransfection with STAT3F. This may reflect an early onset of differentiation which would produce very small clones that would not be scored. Alternatively, very high levels of STAT3F expression may also be toxic, though this has not been reported in other cell types. Morphologically, the differentiated STAT3F colonies closely resembled the differentiated colonies generated on culture of ES cells in the absence of LIF (Figure 7C). Various other cDNAs have been expressed in ES cells using this system, with little or no effect on differentiation (data not shown). This suggested that the effect on differentiation was specifically attributable to expression of STAT3F.

The differentiation induced by expression of STAT3F was examined further by expression analysis of the marker genes *rex1* and *H19*. Rex-1 mRNA, which is specifically expressed in undifferentiated stem cells, was down regulated in STAT3F supertransfectants. In contrast, H19 RNA which is found at low levels in stem cells but is upregulated during differentiation, was increased (Figure 7B). A similar pattern of gene regulation is observed during differentiation of ES cells induced by withdrawal of LIF. These data confirm that the morphological differentiation triggered by STAT3F is accompanied by reprogramming of gene expression.

STAT3F was also expressed from the mouse phosphoglycerate kinase (*pgk-1*) promoter in the episomal vector pHPPGK. This vector gives at least 10-fold lower expression than pHPCAG (data not shown). In this case, there was no significant effect on either colony number or differentiation status of MG 1.19 supertransfectants. A critical level of expression of the dominant interfering mutant therefore appears necessary to block self-renewal.

### Effect of STAT3F on self-renewal is suppressed by co-expression of STAT3

To test whether the induction of differentiation by expression of STAT3F was due to an inhibition of endogenous STAT3 activity, we attempted to rescue the stem cell phenotype by co-expression of wild type STAT3 and also of STAT1 and STAT4. A STAT3F expression vector carrying a blasticidin resistance marker was co-supertransfected into MG1.19 cells with episomal constructs for expression of wild type STATs and hygromycin resistance. Co-supertransfectants were isolated in medium containing both 20µg/ml of blasticidin S and 80µg/ml of hygromycin B. The numbers of stem cell and differentiated colonies were scored after 8 days. As shown in Figure 8, only co-expression of wild type STAT3 restored self-renewal in the presence of STAT3F. Transfection with STAT1 or STAT4 constructs alone had no effect on self-renewal in the absence of STAT3F (not shown) and did not alter differentiation induced by STAT3F. In the case of supertransfection with the CAG promoter STAT1 construct, the total number of colonies (stem + differentiated) recovered was reduced but the relative proportion of stem cell colonies versus differentiated cells was unaltered. This occurred in both the presence and absence of co-expression of STAT3F, and suggests that high level expression of STAT1 may be toxic to ES cells. By using the mouse PGK-1 promoter to drive lower levels of expression comparable numbers of colonies were recovered on transfection with the STAT1 as with the other constructs. In this case, again only the STAT3 construct showed any restoration of stem cell colonies, although to a lower degree than with the high expression CAG vector (not shown). These data indicate that STAT3 has a specific function in ES cells which cannot be compensated by STAT1 or STAT4.

### EXAMPLE 4

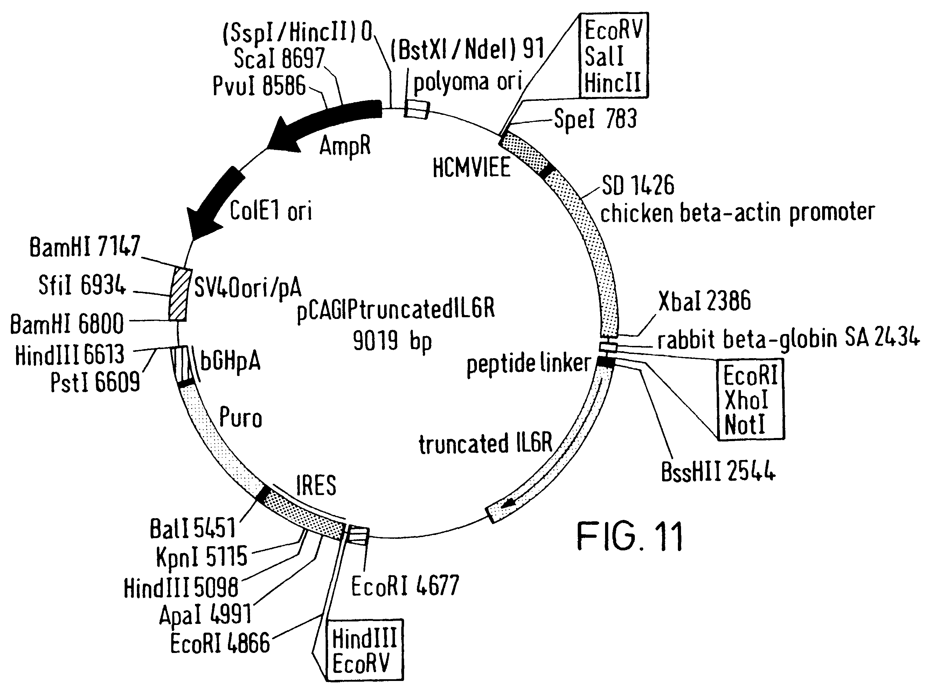

The invention is also used in a strategy for direct selection of genes that code for secreted and cell surface proteins. In one example of this strategy, the basic cloning vector is a truncated form of IL6R that lacks a signal sequence. This vector is described in detail below and shown in Fig. 11. If this truncated IL6R is expressed in ES cells, it is not exported to the cell surface and these cells differentiate when cultured in IL6. However, if the IL6R signal sequence is reconstituted by a signal sequence provided by a cDNA fragments cloned in frame at the 5' end of the truncated IL6R, the chimaeric receptor is expressed on the surface of ES cells. ES cells containing such chimaeric receptors are thus maintained as undifferentiated colonies when cultured in IL6.

Libraries of short, 5' cDNA fragments are produced and cloned into a truncated and modified IL6R-based expression vector. ES cells transformed with such libraries express cDNA:IL6R fusion proteins. However, only cDNAs that encode signal sequences confer IL6 responsiveness on ES cells. These cDNAs alone give rise to undifferentiated, proliferating ES cell clones. This strategy therefore provides a direct selection for cDNAs encoding secreted and cell surface proteins.

The chimaeric IL6R is expressed in the episomal expression system described above (or a derivative thereof). This allows drug selection for episomally transformed cells and high level expression of cloned DNA.

To further refine the selection system, ES cells are modified with two targeted mutations:
a) A selectable marker gene, for example the blasticidin resistance gene, is introduced into the OCT-4 locus by standard targeting techniques. Since Oct-4 is expressed in undifferentiated ES cells, the blasticidin resistance gene will be expressed only by undifferentiated colonies. Blasticidin selection therefore is used to decrease background growth by ensuring rapid deletion of differentiating, Oct-4 negative, ES cells.
b) Since ES cells can produce LIF as an autocrine growth factor, ES cells are used in which both copies of the LIFR gene have been disrupted by gene targeting. This eliminates the possibility of LIF-dependent, false positive colonies that might otherwise persist throughout selection in IL6.

### Details of vector construction:

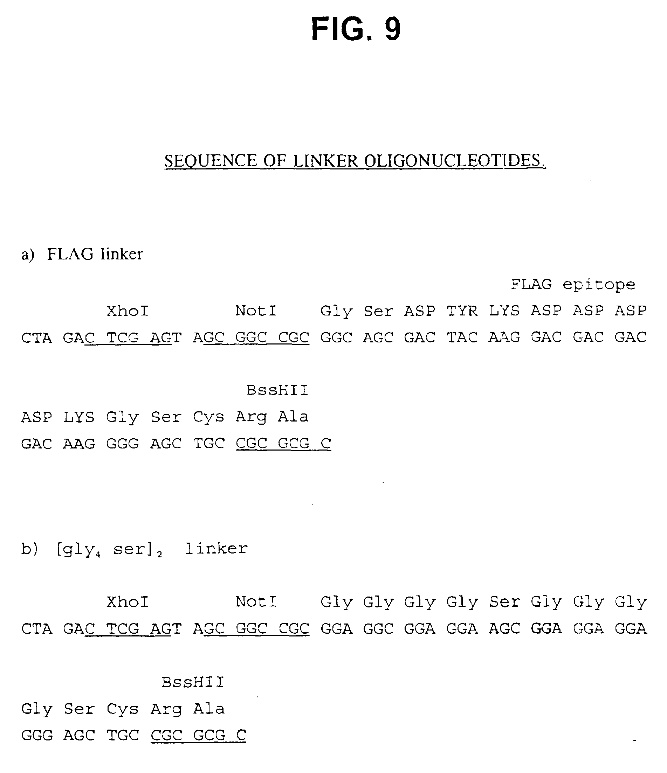

1). IL6R was cloned into the episomal vector pCAGIP or a derivative (pCAGIPXN, i.e. pCAGIP with a destroyed NotI site). pCAGIP contains an internal ribosome entry site (IRES) and a puromycin resistance gene downstream of its multiple cloning site, resulting in stoichiometric production of cDNA:IL6R fusion proteins in transfected cells under puromycin selection. IL6R in pCAGIP provides a positive control (IL6-responsive functional protein on the cell surface), and the basis of the new vector.
2). To construct the cloning vector, IL6R cDNA was truncated by cleavage with BssHII at nucleotide number 92. This deleted the initiator ATG and sequences encoding the signal sequence.
3). To minimise potential steric interference by cloned proteins with IL6 binding and IL6R function, DNA encoding a synthetic flexible linker peptide was then added to the 5' end of the truncated IL6R. Two alternative linkers have been used: gly gly gly gly ser gly gly gly gly ser and a linker containing the FLAG epitope, gly ser ASP TYR LYS ASP ASP ASP ASP LYS (FLAG epitope in upper case). The sequence of these linkers is shown in Fig. 9. In each case, the linker sequence has been cloned in frame with IL6R and has two unique cloning sites (Xhol and Notl) at its 5' end, allowing the introduction of cDNA libraries, or specific cloned sequences, in a directional manner. The FLAG epitope is recognised by a commercially available monoclonal antibody (M2; available from IBI/Kodak) regardless of its position within a fusion protein, and will thus allow the expression levels of surface protein to be measured directly by immunocytochemistry.
4). Vectors containing each of these linkers and an upstream signal sequence are tested for relative expression level and IL6R-function, as detailed below.

To test the utility of these vectors for selecting proteins expressed at the cell surface, a number of known signal sequences are cloned into each vector. These are tested for surface expression and IL6R function. Signal sequences include those from rat CD4 (a protein with extracellular Ig domains), mouse sek (a receptor tyrosine kinase, with no extracellular Ig domains) and mouse sonic hedgehog (a secreted factor).

ES cells are transfected with vectors bearing candidate signal sequences by lipofection or electroporation, followed by puromycin selection for transfected cells. After overnight growth in the presence of LIF, to maintain the undifferentiated state and proliferation, transfected cells are split into three groups and treated with either 1) LIF, 2) IL6 or 3) neither growth factor. Only cells bearing IL6R brought to the cell surface by a fused signal peptide will proliferate in the presence of IL6. Positive controls include ES cells transfected with wild-type IL6R grown in the absence of LIF and the presence of IL6. Negative controls include empty vector (i.e truncated IL6R with no 5' insert) grown in the presence of IL6. To determine whether fusion proteins N-terminal to IL6R block signalling (by steric hindrance), the proportion of such cells that express surface protein but fail to proliferate in response to IL6 is deduced by comparing the number of cells expressing the FLAG epitope with the number that give rise to colonies.

Vectors defined by this assay are then used in cDNA library screens. Preferably, sequences corresponding to 5' ends of cDNAs are generated from full length cDNA libraries and directionally cloned in the screening vector.

In the above description scientific publications are referred to under the following reference numbers:
1. Smith, A. G. (1992) *Seminars in Cell Biology,* **3**, 385-399.
2. Evans, M. J. and Kaufman, M. (1981) *Nature,* **292**, 154-156.
3. Martin, G. R. (1981) *Proc. Natl. Acad. Sci. USA*, **78**, 7634-7638.
4. Doetschman, T. C., Eistetter, H., Katz, M., Schmidt, W. and Kemler, R. (1985) *J.Embryol.Exp.Morphol*., **87**, 27-45.
5. Weiss, M. J. and Orkin, S. H. (1996) *J. Clin. Invest*., **97**, 591-595.
6. Bradley, A., Evans, M. J., Kaufman, M. H. and Robertson, E. (1984) *Nature,* **309**, 255-256.
7. Beddington, R. S. P. and Robertson, E. J. (1989) *Development,* **105**, 733-737.
8. Gassmann, M., Donoho, G., Berg, P. (1995) *Proc.Natl.Acad.Sci.USA*, **92**, 1292-1296.
9. Camenisch, G., Gruber, M., Donoho, G., Van Sloun, P., Wenger, R. and Gassmann, M. (1996) *Nucleic Acids Research,* **24**, 3707-3713.
10. Sambrook, J., Fritsch, E. F. and Maniatis, T. (eds.) (1989) Molecular Cloning: A Laboratory Manual. 2nd ed Ed. 3 vols. Cold Spring Harbor Laboratory Press, Cold Spring Harbor, New York.
11. Niwa, H., Yamamura, K.-I., Miyazaki, J.-I. (1991) *Gene*, **108**, 193-200.
12. Smith, A. G. (1991) *J. Tiss. Cult. Meth*., **13**, 89-94.
13. Beddington, R. S. P., Morgenstern, J., Land, H. and Hogan, A. (1989) *Development*, **106**, 37-46.
14. Hirt, B. J. (1969) *J. Mol. Biol*., **26**, 141-144.
15. te Riele, H., Maandag, E. R., Clarke, A., Hooper, M. and Berns, A. (1990) *Nature*, **348**, 649-651.
16. Fujimura, F. K., Deininger, P. L., Friedmann, T. and Linney, E. (1981) *Cell*, **23**, 809-814.
17. Tsui, L. C., Breitman, M. L., Siminovitch, L. and et al. (1982) *Cell*, **309**, 499-**508**.
18. Smith, A. G. and Rathjen, P. D. (1991) *Sem.Dev.Biol*., **2**, 317-327.
19. Smith, A. G., Heath, J. K., Donaldson, D. D., Wong, G. G., Moreau, J., Stahl, M. and Rogers, D. (1988) *Nature*, **336**, 688-690.
20. Williams, R. L., Hilton, D. J., Pease, S., Willson, T. A., Stewart, C. L., Gearing, D. P., Wagner, E. F., Metcalf, D., Nicola, N. A. and Gough, N. M. (1988) *Nature*, **336**, 684-687.
21. Rathjen, P. D., Nichols, J., Toth, S., Edwards, D. R., Heath, J. K. and Smith, A. G. (1990) *Genes Dev.,* **4,** 2308-2318.
22. Conquet, F., Peyrieras, N., Tiret, L and Brulet, P. (1992) *PNAS*, **89**, 8195-8199.
23. Skarnes, W. C., Auerbach, B. A. and Joyner, A. L. (1992) *Genes Dev*., **6**, 903-918.
24. Friedrich, G. and Soriano, P. (1991) *Genes Dev*., **5**, 1513-1523.
25. Mountford, P., Smith, A.G. (1995) *Trend Genet*., **11**, 179-184.
26. Askew, G. R., Doetschman, T. and Lingrel, J. B. (1993) *MCB*, **13**, 4115-4124.
27. Tucker, K. L., Beard, C., Dausman, J., Jackson-Grusby, L., Laird, P.W., Lei, H., Li, E., Jaenish, R. (1996) *Genes & Dev*., **10**, 1008-1020.
28. Macleod, D., Charlton, J., Mullins, J., Bird, A. (1994) *Genes Dev*., **8**, 2282-2292.
29. Strathdee, C. A., Gavish, H., Shannon, W. R. and Buchwald, M. (1992) *Nature,* **356**, 763-767.
30. Legerski, R. and Peterson, C. (1992) *Nature*, **359**, 70-73.
31. Hayakawa, H., Koike, G., Sekiguchi, M. (1990) J. *Mol. Biol*., **213**, 739-747.
32. Sasaki, K., Watanabe, E., Kawashima, K., Sekine, S., Dohi, T., Oshima, M., Hanai, N., Nishi, T., Hasegawa, M. (1993) *J. Biol. Chem*., **268**, 22782-22787.
33. Nakano, T., Kodama, H., Honjo, T. (1994) *Science*, **265**, 1098-1101.
34. Bain, G., Kitchens, D., Yao, M., Huettner, J.E., Gottlieb, D.I. (1995) *Dev. Biol*., **168**, 342-357.
35. Rohwedel, J., Maltsev, V., Bober, E., Arnold, H.H., Hescheler, J., Wobus, A.M. (1994) *Dev. Biol*., **164**, 87-101.

We have thus described the development of an optimised transfection and expression system which will enable high throughput functional screening of cDNAs in pluripotential mouse embryonic stem (ES) cells and differentiated derivatives. The strategy is based on extrachromosomal vector replication driven by expression of polyoma large T protein. When a vector containing a polyoma origin of replication is introduced into an ES cell line that harbours polyoma large T antigen, a high frequency of stable secondary transfection results. This process is referred to as supertransfection. Supertransfected plasmids can be maintained episomally during long-term culture and during differentiation *in vitro.* Expression of a β-galactosidase reporter from an episomal vector is both ubiquitous and stable, in contrast to the variegated and unstable expression usually observed after cDNA integration into the ES cell genome. Moreover, in the absence of integration, promoter strength is predictable and a range of expression levels can reliably be achieved by using different elements. We also show that episomal vectors can be used for efficient expression of both cytosolic and secreted proteins. These features should make this system invaluable for functional analyses of defined cDNAs and for direct expression screening of cDNA pools or libraries in ES cells.

**Table 1.**

| Comparison of β-galactosidase activities directed by various promoters in transient and stable supertransfectants. | | |
|---|---|---|
| Promoter | Relative β-gal activity | |
| | transient | stable |
| SV40 e/p | 1.0 | 1.0 |
| | | |
| hβAp | 1.1 | 0.7 |
| | | |
| mPGKp | 0.5 | 0.5 |
| | | |
| TKp | 0.1 | 0.1 |
| | | |
| CAG | 19.0 | 1.8 |
| 5x10⁶ MG1.19 ES cells were supertransfected with 20µg of vector DNAs. After 3 days culture for transient expression assay or 8 days selection with hygromycin B for stable expression assay, the β-galactosidase activity generated by these constructs was measured by ONPG assay. Results are normalised relative to activity generated by the SV40e/p construct. See 'Materials and methods' for construction details of vectors. | | |

**Table 2.**

| Supertransfection of LIF and IL-2 expression vectors into MG1.19 ES cells. | | |
|---|---|---|
| Vector | LIF in medium | No. of hyg^{r} stem cell colonies |
| pHPCAG-*lif* | + | 42,000 |
| | | |
| pHPCAG-*lif* | - | 38,000 |
| | | |
| pHPCAG-*il2* | + | 48,000 |
| | | |
| pHPCAG-*il2* | - | 0 |
| 5x10⁶ MG1.19 ES cells were supertransfected with 20µg of vector DNAs. After 8 days selection with 80µg/ml of hygromycin B in the presence or absence of LIF, the number of stem cell colonies were scored. | | |

**Table 3.**

| Efficiency of supertransfection of vectors with various selection markers. | | |
|---|---|---|
| Selection marker | Drug for selection (µg/ml) | No. of resistant colonies |
| PGK*hph*pA | hygromycin B (80) | 50,000 |
| | | |
| SV*bsr*pA | blasticidin S (4) | 12,600 |
| | | |
| hCMV*zeo*pA | zeocin (20) | 20,600 |
| 5x10⁶ MG1.19 ES cells were supertransfected with 20µg of vector DNAs of episomal vectors, pBPCAG and pZPCAG, which carry *bsr* and *zeo* resistance genes respectively. After 8 days selection with the appropriate drug, the number of drug-resistant stem cell colonies were scored. | | |

**Table 4.**

| Effects of overexpression of transgenes in ES cells using pHPCAG. | | |
|---|---|---|
| cDNA | Relative number of hygro^{R} colonies | Colony Size and Morphology |
| None | 1.00 | Normal |
| lacZ | 0.64 | small |
| DIA/LIF | 0.87 | slightly small |
| IL-2 | 0.92 | slightly small |
| Rex-1 | 0.88 | Normal |
| Fgf-2 | 0.65 | Normal |
| Fgf-4 | 0.82 | Normal |
| Fgf-5 | 0.41 | Normal |
| Oct-1 | 0.17 | small |
| Oct-2 | 0.65 | slightly small |
| Oct-3/4 | 0.61 | differentiated |
| Oct-6 | 0.03 | some differentiation |
| c-jun | 0.47 | small |
| E1A | 0.08 | differentiated |
| Jak2 K/E | 0.75 | Normal |
| bcl-2 | 0.28 | small, spindle morphology |
| MAPKP | 1.38 | Normal |
| RXRα | 0.20 | some differentiation |
| RXRβ | 0.63 | Normal |
| RXRγ | 0.91 | Normal |
| COUP-TF1 | 0.40 | some differentiation |
| HNF-4 | 0.05 | Normal |
| Stat1 | 0.10 | small |
| Stat3 | 0.52 | Normal |
| Stat4 | 0.16 | Normal |
| Stat3DON* | 0.14 | differentiated |
| 5x10⁶ ES MG1.19 cells were supertransfected with 20 µg of expression vectors and selected with 80 µg/ml of hygromycin B for 8 days. The numbers of drug-resistant colonies were counted and normalised relative to numbers obtained with empty vector. | | |

| | | |
|---|---|---|
| *Stat3DON is the dominant interfering mutant form of Stat3 described by Akira *et al*. (1996). | | |

## Claims

1. A method of expressing a DNA of interest in a non-human embryonic stem (ES), non-human embryonic carcinoma (EC) or non-human embryonic gonadal (EG) cell, comprising:
(a)
(i) transfecting the cell with a first vector that expresses a replication factor; or
(ii) otherwise obtaining a cell that expresses or will express the replication factor;
(b) transfecting the cell with a second vector, wherein
(i) the second vector contains a DNA coding for a selectable marker in operative combination with a promoter for expression of the selectable marker;
(ii) the second vector additionally contains a second DNA, being the DNA of interest, in operative combination with a promoter for expression of the DNA, and which does not code for a selectable marker;
(iii) extrachromosomal replication of the second vector is dependent upon the presence within the cell of the replication factor; and
(c) expressing the second DNA.

2. A method according to Claim 1 wherein the replication factor is a viral replication factor.

3. A method according to Claim 1 or 2 wherein the viral replication factor is selected from polyoma large T antigen, EBNA-1 antigen, papilloma virus replication factors, SV40 large T antigen and functional variants, analogues and derivatives thereof appropriate to the cell species.

4. A method according to any of Claims 1-3 wherein the second vector does not express the replication factor.

5. A method according to any of Claims 1-4, wherein the second vector is substantially free of DNA coding for the replication factor or any part thereof.

6. A method according to any of Claims 1-5 wherein the selectable marker is an antibiotic resistance gene.

7. A method according to any of Claims 1-6 further comprising transfecting the cell with a third vector, wherein the third vector contains a DNA, or is adapted to receive a DNA, in operative combination with a promoter for expression of the DNA, and replication of the third vector is dependant upon presence within the cell of the replication factor.

8. A method according to Claim 7 wherein the third vector expresses a selectable marker, which selectable marker is different to that expressed by the second vector.

9. A method according to any preceding claim wherein the cell is a non-human mammalian cell or an avian cell.

10. A method according to any preceding claim wherein the cell is a mouse cell.

11. A method according to any preceding claim wherein the cell is a non-human ES cell.

12. A method according to any preceding claim for transfection of a non-human ES cell wherein the ES cell of step (a) expresses polyoma large T antigen and the second vector comprises a natural target for polyoma large T antigen, such as *Ori* or functional variants thereof adapted to bind to polyoma large T antigen.

13. A method according to any preceding claim wherein the DNA codes for a polypeptide or protein.

14. A method according to any of Claims 1-12 wherein the DNA codes for an antisense RNA.

15. A method according to any preceding claims wherein the promoter is inducible.

16. A method according to any preceding claim wherein transcription of the DNA can be activated by a site specific recombinase.

17. A method according to any preceding claim wherein replication of the second vector can be prevented by a site specific recombinase.

18. A method according to any preceding claim wherein the DNA is a cDNA.

19. A method according to any preceding claim wherein the second vector is constructed from a vector containing DNA encoding the replication factor wherein during construction the DNA encoding the replication factor is deleted.

20. A method according to any preceding claim, comprising carrying out step (a)(i) to obtain transfected cells, dividing the transfected cells into separate groups and carrying out steps (b) and (c) on the separate groups using second vectors containing different DNAs of interest.

21. A vector for transfection of an ES, EC or EG cell so as to express a DNA of interest, wherein:
(i) the vector contains a DNA coding for a selectable marker in operative combination with a promoter for expression of the selectable marker;
(ii) the vector contains a second DNA, being the DNA of interest, in operative combination with a promoter for expression of the DNA, and which does not code for a selectable marker;
(iii) extrachromosomal replication of the vector is dependent upon presence within the cell of a replication factor; and
(iv) the vector does not express the replication factor.

22. A vector according to Claim 21 wherein the replication factor is a viral replication factor.

23. A vector according to Claim 21 or 22 wherein the viral replication factor is selected from polyoma large T antigen, EBNA-1 antigen, papilloma virus replication factors, SV40 large T antigen and functional variants, analogues and derivatives thereof.

24. A vector according to any of Claims 21 to 23 wherein the vector is substantially free of DNA coding for the replication factor or any part thereof.

25. A vector according to any of Claims 21 to 24 for transfection of mammalian or avian cells.

26. A vector according to any of Claims 21 to 25 for transfection of ES cells.

27. A vector according to Claim 26 comprising a natural target for polyoma large T antigen, such as *Ori* or functional variants thereof adapted to bind to polyoma large T antigen. ,

28. A vector according to any of Claims 21-27 wherein the DNA codes for a polypeptide or protein.

29. A vector according to any of Claims 21-27 wherein the DNA codes for an antisense DNA.

30. A vector according to any of Claims 21-29 wherein the promoter is inducible.

31. A vector according to any of Claims 21-30 wherein the selectable marker is an antibiotic resistance gene.

32. A vector according to any of Claims 21-31, wherein the second DNA is a cDNA.

33. Use of a vector according to any of Claims 21-32 for expression of a DNA sequence within a non-human cell.

34. A non-human ES, EC or EG cell transfected with a first vector that expresses a replication factor and with a second vector according to any of Claims 21-32.

35. A non-human mammalian cell according to Claim 34.

36. A non-human ES cell according to Claim 34.

37. A mouse cell according to Claim 34.

38. An avian cell according to Claim 34.

39. A cell selected from a non-human ES, EC or EG cell according to any of Claims 34 to 38, and differentiated progeny thereof.

40. An assay for the effect of presence in a non-human ES, EC or EG cell of a protein or polypeptide or other product of DNA expression comprising the steps of the method of any of Claims 1-20 and further comprising:
(a) selecting for cells that have been transfected with the second vector; and
(b) maintaining the selected cells over a plurality of generations so as to assay the effect of expression of the protein or polypeptide or other product of DNA expression.

41. An assay according to Claim 40 wherein step (a) is carried out once and the cells obtained are divided and used for a plurality of separate assays in which steps (b)-(d) are carried out a plurality of times with second vectors containing different DNA sequences.

42. An assay according to Claim 40 or 41 for assay of the effect of presence in the cell of two factors, each factor being independently selected from a protein, a polypeptide and another product of DNA expression.

43. A method of screening a library of cDNAs comprising assaying the effect of expression of each of the cDNAs according to the method of any of Claims 40-42.

44. A method according to Claim 1 for investigating the properties of a DNA sequence comprising expressing in a non-human ES, EC or EG a cell a composite DNA including (a) the DNA sequence under investigation, linked to (b) a DNA coding for a cell active protein, wherein
activity of the cell active protein is dependant upon transport of the cell active protein to the cell surface, and
the DNA of (b) does not code for a polypeptide capable of directing transportation of the cell active protein to the cell surface, wherein the composite DNA is expressed by the method of any of Claims 1-20, and further comprising:
(a) selecting for cells that have been transfected with the second vector; and
(b) maintaining the selected cells over a plurality of generations so as to assay the effect of expression of the composite DNA.

45. A method according to Claim 44 for screening a library of DNAs to identify DNA sequences coding for signal polypeptide sequences that transport proteins to the cell surface, and the method optionally comprises determining whether the cell active protein is transported to the cell surface and remains there or is secreted by the cell.

46. A method according to Claim 44 or 45 wherein the DNA of (b) is obtained by deleting or disabling, from a DNA encoding a cell surface or secreted protein, that portion of the DNA that codes for the polypeptide sequence responsible for transportation of the protein to the cell surface.

47. A method according to any of Claims 44-46 wherein the cell active protein induces a morphological or proliferative change in the cell.

48. A method according to any of Claims 44-47 wherein the cell active protein inhibits differentiation of the cell and in the absence of the cell active protein the cell will differentiate.

49. A method according to any of Claims 44-48 wherein the cell active protein is a cell surface receptor.

50. A method according to Claim 49 wherein the cell active protein is an IL-6 receptor and the DNA of (b) encodes a modified form of the receptor preprotein lacking a functional signal sequence.

51. A method according to any of Claims 44-50 comprising investigating the properties of a DNA in mammalian or avian cells.

52. A method according to any of Claims 44-51 comprising investigating the properties of a DNA in embryonic cells.

53. A method according to Claim 52 comprising investigating the properties of a DNA in non-human ES, EC or EG cells or differentiated progeny of such cells.

54. A method according to any of Claims 44-53 wherein step (a) is carried out once and the cells obtained are divided and used for a plurality of separate methods in which steps (b)-(d) are carried out a plurality of times with second vectors containing different DNA sequences.

55. A method according to any of Claims 44-54 for identification of a DNA coding for a cell surface or secreted protein.

56. A method according to any of Claims 44-55 for identification of a cell surface or secreted protein.

## Patentansprüche

1. Verfahren zum Exprimieren einer DNA, die von Interesse ist, in einer nicht-menschlichen embryonalen Stamm- (ES), nicht-menschlichen embryonalen Krebs- (EC) oder nicht-menschlichen embryonalen Gonaden- (EG) Zelle, umfassend:
(a)
(i) Transfektieren der Zelle mit einem ersten Vektor, der einen Replikationsfaktor exprimiert; oder
(ii) anderweitiges Erhalten einer Zelle, die den Replikationsfaktor exprimiert oder exprimieren wird;
(b) Transfektieren der Zelle mit einem zweiten Vektor, wobei
(i) der zweite Vektor eine DNA enthält, die für einen selektierbaren Marker in einer funktionsfähigen Kombination mit einem Promotor zur Expression des selektierbaren Markers codiert;
(ii) der zweite Vektor zusätzlich eine zweite DNA enthält, bei der es sich um die DNA, die von Interesse ist, handelt, in einer funktionsfähigen Kombination mit einem Promotor zur Expression der DNA und die nicht für einen selektierbaren Marker codiert;
(iii) die extrachromosomale Replikation des zweiten Vektors von der Gegenwart des Replikationsfaktors innerhalb der Zelle abhängig ist; und
(c) Exprimieren der zweiten DNA.

2. Verfahren nach Anspruch 1, wobei es sich bei dem Replikationsfaktor um einen viralen Replikationsfaktor handelt.

3. Verfahren nach Anspruch 1 oder 2, wobei der virale Replikationsfaktor aus Polyoma large T Antigen, EBNA-1 Antigen, Papillomavirus-Replikationsfaktoren, SV40 large T Antigen und funktionellen Varianten, Analogons und Derivaten davon, die für die Zellart geeignet sind, ausgewählt ist.

4. Verfahren nach einem der Ansprüche 1-3, wobei der zweite Vektor den Replikationsfaktor nicht exprimiert.

5. Verfahren nach einem der Ansprüche 1-4, wobei der zweite Vektor im Wesentlichen frei von DNA ist, die für den Replikationsfaktor oder einen beliebigen Teil davon codiert.

6. Verfahren nach einem der Ansprüche 1-5, wobei es sich bei dem selektierbaren Marker um ein Antibiotika-Resistenzgen handelt.

7. Verfahren nach einem der Ansprüche 1-6, das ferner Transfektieren der Zelle mit einem dritten Vektor, wobei der dritte Vektor eine DNA enthält oder angepasst ist, eine DNA zu empfangen, in funktionsfähiger Kombination mit einer Promotor zur Expression der DNA umfasst und Replikation des dritten Vektors von der Gegenwart des Replikationsfaktors innerhalb der Zelle abhängig ist.

8. Verfahren nach Anspruch 7, wobei der dritte Vektor einen selektierbaren Marker exprimiert, wobei der selektierbare Marker von dem, der durch den zweiten Vektor exprimiert wird, verschieden ist.

9. Verfahren nach einem vorhergehenden Anspruch, wobei es sich bei der Zelle um eine nicht-menschliche Säugetierzelle oder eine Vogelzelle handelt.

10. Verfahren nach einem vorhergehenden Anspruch, wobei es sich bei der Zelle um eine Mauszelle handelt.

11. Verfahren nach einem vorhergehenden Anspruch, wobei es sich bei der Zelle um eine nicht-menschliche ES-Zelle handelt.

12. Verfahren nach einem vorhergehenden Anspruch zur Transfektion einer nicht-menschlichen ES-Zelle, wobei die ES-Zelle von Schritt (a) Polyoma large T Antigen exprimiert und der zweite Vektor ein natürliches Ziel für Polyoma large T Antigen umfasst, wie *Ori* oder funktionelle Varianten davon, die angepasst sind, Polyoma large T Antigen zu binden.

13. Verfahren nach einem vorhergehenden Anspruch, wobei die DNA für ein Polypeptid oder Protein codiert.

14. Verfahren nach einem der Ansprüche 1-12, wobei die DNA für eine Antisense-RNA codiert.

15. Verfahren nach einem der vorhergehenden Ansprüche, wobei der Promotor induzierbar ist.

16. Verfahren nach einem vorhergehenden Anspruch, wobei Transkription der DNA durch eine ortsspezifische Rekombinase aktiviert werden kann.

17. Verfahren nach einem vorhergehenden Anspruch, wobei Replikation des zweiten Vektors durch eine ortsspezifische Rekombinase verhindert werden kann.

18. Verfahren nach einem vorhergehenden Anspruch, wobei es sich bei der DNA um eine cDNA handelt.

19. Verfahren nach einem vorhergehenden Anspruch, wobei der zweite Vektor aus einem Vektor, der DNA enthält, die den Replikationsfaktor codiert, aufgebaut ist, wobei während des Aufbaus die DNA, die den Replikationsfaktor codiert, entfernt wird.

20. Verfahren nach einem vorhergehenden Anspruch, das Ausführen von Schritt (a)(i), um transfektierte Zellen zu erhalten, Aufteilen der transfektierten Zellen in separate Gruppen und Ausführen der Schritte (b) und (c) an den separaten Gruppen unter Verwendung von zweiten Vektoren, die verschiedene DNAs, die von Interesse sind, enthalten, umfasst.

21. Vektor zur Transfektion einer ES-, EC- oder EG-Zelle, um eine DNA, die von Interesse ist, zu exprimieren, wobei:
(i) der Vektor eine DNA enthält, die für einen selektierbaren Marker in funktionsfähiger Kombination mit einem Promotor zur Expression des selektierbaren Markers codiert;
(ii) der Vektor eine zweite DNA, bei der es sich um die DNA, die von Interesse ist, handelt, in funktionsfähiger Kombination mit einem Promotor zur Expression der DNA enthält und die nicht für einen selektierbaren Marker codiert;
(iii) die extrachromosomale Replikation des Vektors von der Gegenwart eines Replikationsfaktors innerhalb der Zelle abhängig ist; und
(iv) der Vektor den Replikationsfaktor nicht exprimiert.

22. Vektor nach Anspruch 21, wobei es sich bei dem Replikationsfaktor um einen viralen Replikationsfaktor handelt.

23. Vektor nach Anspruch 21 oder 22, wobei der virale Replikationsfaktor aus Polyoma large T Antigen, EBNA-1 Antigen, Papillomavirus-Replikationsfaktoren, SV40 large T Antigen und funktionellen Varianten, Analogons und Derivaten davon ausgewählt ist.

24. Vektor nach einem der Ansprüche 21 bis 23, wobei der Vektor im Wesentlichen frei von DNA ist, die für den Replikationsfaktor oder einen beliebigen Teil davon codiert.

25. Vektor nach einem der Ansprüche 21 bis 24 zur Transfektion von Säugetier- oder Vogelzellen.

26. Vektor nach einem der Ansprüche 21 bis 25 zur Transfektion von ES-Zellen.

27. Vektor nach Anspruch 26, der ein natürliches Ziel für Polyoma large T Antigen umfasst, wie *Ori* oder funktionelle Varianten davon, die angepasst sind, um Polyoma large T Antigen zu binden.

28. Vektor nach einem der Ansprüche 21-27, wobei die DNA für ein Polypeptid oder Protein codiert.

29. Vektor nach einem der Ansprüche 21-27, wobei die DNA für eine Antisense-DNA codiert.

30. Vektor nach einem der Ansprüche 21-29, wobei der Promotor induzierbar ist.

31. Vektor nach einem der Ansprüche 21-30, wobei es sich bei dem selektierbaren Marker um ein Antibiotika-Resistenzgen handelt.

32. Vektor nach einem der Ansprüche 21-31, wobei es sich bei der zweiten DNA um eine cDNA handelt.

33. Verwendung eines Vektors nach einem der Ansprüche 21-32 zur Expression einer DNA-Sequenz innerhalb einer nicht-menschlichen Zelle.

34. Nicht-menschliche ES-, EC- oder EG-Zelle, die mit einem ersten Vektor, der einen Replikationsfaktor exprimiert, und mit einem zweiten Vektor nach einem der Ansprüche 21-32 transfektiert ist.

35. Nicht-menschliche Säugetierzelle nach Anspruch 34.

36. Nicht-menschliche ES-Zelle nach Anspruch 34.

37. Mauszelle nach Anspruch 34.

38. Vogelzelle nach Anspruch 34.

39. Zelle, die aus einer nicht-menschlichen ES-, EC- oder EG-Zelle nach einem der Ansprüche 34 bis 38 ausgewählt ist, und differenzierte Nachkommen davon.

40. Analyse zum Effekt der Gegenwart eines Proteins oder Polypeptids oder anderen Produkts von DNA-Expression in einer nicht-menschlichen ES-, EC- oder EG-Zelle, umfassend die Schritte des Verfahrens nach einem der Ansprüche 1-20 und ferner umfassend:
(a) Selektieren von Zellen, die mit dem zweiten Vektor transfektiert worden sind; und
(b) Aufrechterhalten der selektierten Zellen über mehrere Generationen, um den Effekt der Expression des Proteins oder Polypeptids oder anderen Produkts von DNA-Expression zu analysieren.

41. Analyse nach Anspruch 40, wobei Schritt (a) einmal ausgeführt wird und die erhaltenen Zellen aufgeteilt und für mehrere separate Analysen verwendet werden, in denen die Schritte (b)-(d) mehrere Male mit zweiten Vektoren, die verschiedene DNA-Sequenzen enthalten, ausgeführt werden.

42. Analyse nach Anspruch 40 oder 41 zur Analyse des Effekts der Gegenwart von zwei Faktoren in der Zelle, wobei jeder Faktor unabhängig voneinander aus einem Protein, Polypeptid und anderen Produkt von DNA-Expression ausgewählt ist.

43. Verfahren zur Selektion einer Bibliothek von cDNAs, das Analysieren des Effekts der Expression von jeder der cDNAs nach dem Verfahren nach einem der Ansprüche 40-42 umfasst.

44. Verfahren nach Anspruch 1 zum Untersuchen der Eigenschaften einer DNA-Sequenz, welches das Exprimieren in einer nicht-menschlichen ES-, EC- oder EG-Zelle einer zusammengesetzten DNA umfasst, die (a) die DNA-Sequenz, die untersucht wird, verbunden mit (b) einer DNA, die für ein zellaktives Protein codiert, einschließt, wobei
die Aktivität des zellaktiven Proteins vom Transport des zellaktiven Proteins zu der Zelloberfläche abhängt, und
die DNA von (b) nicht für ein Polypeptid, das in der Lage ist, den Transport des zellaktiven Proteins zu der Zelloberfläche zu regeln, codiert, wobei die zusammengesetzte DNA durch das Verfahren nach einem der Ansprüche 1-20 exprimiert wird, und ferner umfassend:
(a) Selektieren von Zellen, die mit dem zweiten Vektor transfektiert worden sind; und
(b) Aufrechterhalten der selektierten Zellen über mehrere Generationen, um den Effekt der Expression der zusammengesetzten DNA zu analysieren.

45. Verfahren nach Anspruch 44 zur Selektion einer Bibliothek von DNAs, um DNA-Sequenzen zu identifizieren, die für Signal-Polypeptid-Sequenzen codieren, die Proteine zu der Zelloberfläche transportieren, wobei das Verfahren gegebenenfalls Bestimmen, ob das zellaktive Protein zu der Zelloberfläche transportiert wird und dort verbleibt oder von der Zelle sezerniert wird, umfasst.

46. Verfahren nach Anspruch 44 oder 45, wobei die DNA von (b) durch Entfernen oder Inaktivieren von einer DNA, die ein Zelloberflächen- oder ein sezerniertes Protein codiert, den Teil der DNA, der für die Polypeptidsequenz, die für den Transport des Proteins zu der Zelloberfläche verantwortlich ist, codiert.

47. Verfahren nach einem der Ansprüche 44-46, wobei das zellaktive Protein in der Zelle eine morphologische oder proliferative Veränderung in der Zelle induziert.

48. Verfahren nach einem der Ansprüche 44-47, wobei das zellaktive Protein Differenzierung der Zelle inhibiert und in Abwesenheit des zellaktiven Proteins die Zelle sich differenziert.

49. Verfahren nach einem der Ansprüche 44-48, wobei es sich bei dem zellaktiven Protein um einen Zelloberflächenrezeptor handelt.

50. Verfahren nach Anspruch 49, wobei es sich bei dem zellaktiven Protein um einen IL-6 Rezeptor handelt und die DNA von (b) eine modifizierte Form des Rezeptor-Präproteins, dem eine funktionelle Signalsequenz fehlt, codiert.

51. Verfahren nach einem der Ansprüche 44-50, das Untersuchen der Eigenschaften einer DNA in Säugetier- oder Vogelzellen umfasst.

52. Verfahren nach einem der Ansprüche 44-51, das Untersuchen der Eigenschaften einer DNA in embryonalen Zellen umfasst.

53. Verfahren nach Anspruch 52, das Untersuchen der Eigenschaften einer DNA in nicht-menschlichen ES-, EC- oder EG-Zellen oder differenzierten Nachkommen solcher Zellen umfasst.

54. Verfahren nach einem der Ansprüche 44-53, wobei Schritt (a) einmal ausgeführt wird und die erhaltenen Zellen aufgeteilt und für mehrere separate Verfahren verwendet werden, in denen die Schritte (b)-(d) mehrere Male mit zweiten Vektoren, die verschiedene DNA-Sequenzen enthalten, ausgeführt werden.

55. Verfahren nach einem der Ansprüche 44-54 zur Identifizierung einer DNA, die für ein Zelloberflächen- oder ein sezerniertes Protein codiert.

56. Verfahren nach einem der Ansprüche 44-55 zur Identifizierung eines Zelloberflächen- oder eines sezernierten Proteins.

## Revendications

1. Procédé pour exprimer un ADN d'intérêt dans une cellule souche embryonnaire (ES) non humaine, de carcinome embryonnaire (EC) non humaine ou gonadique embryonnaire (EG) non humaine, comprenant :
(a)
(i) de transfecter la cellule avec un premier vecteur qui exprime un facteur de réplication ; ou
(ii) d'obtenir de toute autre manière une cellule qui exprime ou exprimera le facteur de réplication ;
(b) de transfecter la cellule avec un second vecteur, dans lequel
(i) le second vecteur contient un ADN codant pour un marqueur sélectionnable en combinaison opérationnelle avec un promoteur pour l'expression du marqueur sélectionnable ;
(ii) le second vecteur contient en outre un second ADN, étant l'ADN d'intérêt, en combinaison opérationnelle avec un promoteur pour l'expression de l'ADN, et qui ne code pas pour un marqueur sélectionnable ;
(iii) la réplication extrachromosomique du second vecteur est dépendante de la présence dans la cellule du facteur de réplication ; et
(c) d'exprimer le second ADN.

2. Procédé selon la revendication 1, dans lequel le facteur de réplication est un facteur de réplication viral.

3. Procédé selon la revendication 1 ou 2, dans lequel le facteur de réplication viral est choisi parmi l'antigène grand T du virus du polyome, l'antigène EBNA-1, les facteurs de réplication du virus du papillome, l'antigène grand T de SV40 et les variantes, analogues et dérivés fonctionnels de ceux-ci appropriés à l'espèce cellulaire.

4. Procédé selon l'une quelconque des revendications 1 à 3, dans lequel le second vecteur n'exprime pas le facteur de réplication.

5. Procédé selon l'une quelconque des revendications 1 à 4, dans lequel le second vecteur est substantiellement exempt d'ADN codant pour le facteur de réplication ou une partie quelconque de celui-ci.

6. Procédé selon l'une quelconque des revendications 1 à 5, dans lequel le marqueur sélectionnable est un gène de résistance à un antibiotique.

7. Procédé selon l'une quelconque des revendications 1 à 6, comprenant en outre de transfecter la cellule avec un troisième vecteur, dans lequel le troisième vecteur contient un ADN, ou est approprié pour recévoir un ADN, en combinaison opérationnelle avec un promoteur pour l'expression de l'ADN, et la réplication du troisième vecteur est dépendante de la présence dans la cellule du facteur de réplication.

8. Procédé selon la revendication 7, dans lequel le troisième vecteur exprime un marqueur sélectionnable, lequel marqueur sélectionnable est différent de celui exprimé par le second vecteur.

9. Procédé selon l'une quelconque des revendications précédentes dans lequel la cellule est une cellule mammifère non humaine ou une cellule aviaire.

10. Procédé selon l'une quelconque des revendications précédentes dans lequel la cellule est une cellule de souris.

11. Procédé selon l'une quelconque des revendications précédentes dans lequel la cellule est une cellule ES non humaine.

12. Procédé selon l'une quelconque des revendications précédentes pour la transfection d'une cellule ES non humaine dans lequel la cellule ES de l'étape (a) exprime l'antigène grand T du virus du polyome et le second vecteur comprend une cible naturelle pour l'antigène grand T du virus du polyome, telle que *Ori* ou des variantes fonctionnelles de celui-ci appropriées pour se lier à l'antigène grand T du virus du polyome.

13. Procédé selon l'une quelconque des revendications précédentes, dans lequel l'ADN code pour un polypeptide ou une protéine.

14. Procédé selon l'une quelconque des revendications 1 à 12, dans lequel l'ADN code pour un ARN antisens.

15. Procédé selon l'une quelconque des revendications précédentes, dans lequel le promoteur est inductible.

16. Procédé selon l'une quelconque des revendications précédentes, dans lequel la transcription de l'ADN peut être activée par une recombinase spécifique au site.

17. Procédé selon l'une quelconque des revendications précédentes, dans lequel la réplication du second vecteur peut être empêchée par une recombinase spécifique au site.

18. Procédé selon l'une quelconque des revendications précédentes, dans lequel l'ADN est un ADNc.

19. Procédé selon l'une quelconque des revendications précédentes, dans lequel le second vecteur est construit à partir d'un vecteur contenant l'ADN codant pour le facteur de réplication dans lequel pendant la construction l'ADN codant pour le facteur de réplication subit une délétion.

20. Procédé selon l'une quelconque des revendications précédentes, comprenant d'exécuter l'étape (a)(i) pour obtenir des cellules transfectées, de diviser les cellules transfectées en groupes séparés et d'exécuter les étapes (b) et (c) sur les groupes séparés en utilisant des seconds vecteurs contenant différents ADN d'intérêt.

21. Vecteur pour la transfection d'une cellule ES, EC ou EG de manière à exprimer un ADN d'intérêt, dans lequel :
(i) le vecteur contient un ADN codant pour un marqueur sélectionnable en combinaison opérationnelle avec un promoteur pour l'expression du marqueur sélectionnable ;
(ii) le vecteur contient un second ADN, étant l'ADN d'intérêt, en combinaison opérationnelle avec un promoteur pour l'expression de l'ADN, et qui ne code pas pour un marqueur sélectionnable ;
(iii) la réplication extrachromosomique du vecteur dépend de la présence dans la cellule d'un facteur de réplication ; et
(iv) le vecteur n'exprime pas le facteur de réplication.

22. Vecteur selon la revendication 21, dans lequel le facteur de réplication est un facteur de réplication viral.

23. Vecteur selon la revendication 21 ou 22, dans lequel le facteur de réplication viral est choisi parmi l'antigène grand T du virus du polyome, l'antigène EBNA-1, les facteurs de réplication du virus du papillome, l'antigène grand T de SV40 et les variantes, analogues et dérivés fonctionnels de ceux-ci.

24. Vecteur selon l'une quelconque des revendications 21 à 23, dans lequel le vecteur est substantiellement exempt d'ADN codant pour le facteur de réplication ou une partie quelconque de celui-ci.

25. Vecteur selon l'une quelconque des revendications 21 à 24 pour la transfection de cellules mammifères ou aviaires.

26. Vecteur selon l'une quelconque des revendications 21 à 25 pour la transfection de cellules ES.

27. Vecteur selon la revendication 26, comprenant une cible naturelle pour l'antigène grand T du virus du polyome, telle que *Ori* ou des variantes fonctionnelles de celui-ci appropriées pour se lier à l'antigène grand T du virus du polyome.

28. Vecteur selon l'une quelconque des revendications 21 à 27 dans lequel l'ADN code pour un polypeptide ou une protéine.

29. Vecteur selon l'une quelconque des revendications 21 à 27 dans lequel l'ADN code pour un ADN antisens.

30. Vecteur selon l'une quelconque des revendications 21 à 29 dans lequel le promoteur est inductible.

31. Vecteur selon l'une quelconque des revendications 21 à 30 dans lequel le marqueur sélectionnable est un gène de résistance à un antibiotique.

32. Vecteur selon l'une quelconque des revendications 21 à 31 dans lequel le second ADN est un ADNc.

33. Utilisation d'un vecteur selon l'une quelconque des revendications 21 à 32 pour l'expression d'une séquence d'ADN dans une cellule non humaine.

34. Cellule ES, EC ou EG non humaine transfectée avec un premier vecteur qui exprime un facteur de réplication et avec un second vecteur selon l'une quelconque des revendications 21 à 32.

35. Cellule mammifère non humaine selon la revendication 34.

36. Cellule ES non humaine selon la revendication 34.

37. Cellule de souris selon la revendication 34.

38. Cellule aviaire selon la revendication 34.

39. Cellule choisie parmi une cellule ES, EC ou EG non humaine selon l'une quelconque des revendications 34 à 38, et progéniture différenciée de celle-ci.

40. Dosage pour l'effet de la présence dans une cellule ES, EC ou EG non humaine d'une protéine ou d'un polypeptide ou autre produit d'expression d'ADN comprenant les étapes du procédé selon l'une quelconque des revendications 1 à 20 et comprenant en outre :
(a) de sélectionner les cellules qui ont été transfectées avec le second vecteur ; et
(b) de conserver les cellules sélectionnées sur une pluralité de générations de manière à doser l'effet de l'expression de la protéine ou du polypeptide ou autre produit d'expression d'ADN.

41. Dosage selon la revendication 40, dans lequel l'étape (a) est exécutée une fois et les cellules obtenues sont divisées et utilisées pour une pluralité de dosages séparés dans lesquels les étapes (b) à (d) sont exécutées une pluralité de fois avec des seconds vecteurs contenant des séquences d'ADN différentes.

42. Dosage selon la revendication 40 ou 41, pour le dosage de l'effet de la présence dans la cellule de deux facteurs, chaque facteur étant indépendamment choisi parmi une protéine, un polypeptide et un autre produit d'expression d'ADN.

43. Procédé de criblage d'une librairie d'ADNc comprenant de doser l'effet de l'expression de chacun des ADNc selon le procédé de l'une quelconque des revendications 40 à 42.

44. Procédé selon la revendication 1 pour effectuer des recherches sur les propriétés d'une séquence d'ADN comprenant d'exprimer dans une cellule ES, EC ou EG non humaine un ADN composite incluant (a) la séquence d'ADN objet des recherches, liée à (b) un ADN codant pour une protéine active cellulaire, dans lequel
l'activité de la protéine active cellulaire est dépendante du transport de la protéine active cellulaire à la surface de la cellule, et
l'ADN de (b) ne code pas pour un polypeptide capable de diriger le transport de la protéine active cellulaire à la surface de la cellule, dans lequel l'ADN composite est exprimé par le procédé de l'une quelconque des revendications 1 à 20, et comprenant en outre :
(a) de sélectionner les cellules qui ont été transfectées avec le second vecteur ; et
(b) de conserver les cellules sélectionnées sur une pluralité de générations de manière à doser l'effet de l'expression de l'ADN composite.

45. Procédé selon la revendication 44 pour cribler une librairie d'ADN pour identifier les séquences d'ADN codant pour les séquences de polypeptides signal qui transportent les protéines à la surface cellulaire, et le procédé comprend facultativement de déterminer si la protéine active cellulaire est transportée à la surface cellulaire et y reste ou est sécrétée par la cellule.

46. Procédé selon la revendication 44 ou 45 dans lequel l'ADN de (b) est obtenu par délétion ou désactivation, à partir d'un ADN codant pour une protéine de surface cellulaire ou sécrétée, de la partie de l'ADN qui code pour la séquence de polypeptide responsable du transport de la protéine à la surface cellulaire.

47. Procédé selon l'une quelconque des revendications 44 à 46, dans lequel la protéine active cellulaire induit un changement morphologique ou prolifératif dans la cellule.

48. Procédé selon l'une quelconque des revendications 44 à 47, dans lequel la protéine active cellulaire inhibe la différentiation de la cellule et en l'absence de la protéine active cellulaire la cellule se différenciera.

49. Procédé selon l'une quelconque des revendications 44 à 48, dans lequel la protéine active cellulaire est un récepteur à la surface cellulaire.

50. Procédé selon la revendication 49, dans lequel la protéine active cellulaire est un récepteur IL-6 et l'ADN de (b) code pour une forme modifiée de la pré-protéine du récepteur à laquelle manque une séquence signal fonctionnelle.

51. Procédé selon l'une quelconque des revendications 44 à 50, comprenant d'effectuer des recherches sur les propriétés d'un ADN dans des cellules mammifères ou aviaires.

52. Procédé selon l'une quelconque des revendications 44 à 51, comprenant d'effectuer des recherches sur les propriétés d'un ADN dans des cellules embryonnaires.

53. Procédé selon la revendication 52, comprenant d'effectuer des recherches sur les propriétés d'un ADN dans des cellules ES, EC ou EG non humaines ou la progéniture différenciée de telles cellules.

54. Procédé selon l'une quelconque des revendications 44 à 53, dans lequel l'étape (a) est exécutée une fois et les cellules obtenues sont divisées et utilisées pour une pluralité de procédés séparés dans lesquels les étapes (b) à (d) sont exécutées une pluralité de fois avec des seconds vecteurs contenant des séquences d'ADN différentes.

55. Procédé selon l'une quelconque des revendications 44 à 54 pour l'identification d'un ADN codant pour une protéine de surface cellulaire ou sécrétée.

56. Procédé selon l'une quelconque des revendications 44 à 55 pour l'identification d'une protéine de surface cellulaire ou sécrétée.
